# EUROPEAN PATENT APPLICATION

(11) **EP 4 342 487 A2**
(43) Date of publication of application: **27.03.2024**
(21) Application number: 22746248.8
(22) Date of filing: 27.01.2022
(51) Int. Cl.: A61K 38/22, A61K 47/68, A61P 11/00, A61P 31/14, C07K 14/72, C07K 19/00

(54) **PHARMACEUTICAL COMPOSITION COMPRISING GIP DERIVATIVE OR LONG-ACTING CONJUGATE THEREOF FOR PREVENTING OR TREATING PULMONARY DISEASE**

(30) Priority: 29.01.2021 KR 20210013179
(71) Applicant: Hanmi Pharm. Co., Ltd., Hwaseong-si, Gyeonggi-do 18536 (KR)
(72) Inventor: CHOI, Jae Hyuk, Hwaseong-si, Gyeonggi-do 18469 (KR); KIM, Eun Jung, Hwaseong-si, Gyeonggi-do 18469 (KR); KIM, Won Ki, Hwaseong-si, Gyeonggi-do 18469 (KR); HAN, Seung Su, Hwaseong-si, Gyeonggi-do 18469 (KR); SHIN, Min Kyung, Hwaseong-si, Gyeonggi-do 18469 (KR)
(74) Representative: Brevalex
(86) International application number: PCT/KR2022/001472
(87) International publication number: WO 2022/164222

(57) **Abstract**

Provided is a pharmaceutical composition for preventing or treating lung disease, including a GIP derivative, a pharmaceutically acceptable salt thereof, a solvate thereof, or a conjugate thereof.

## Description

### TECHNICAL FIELD

The present disclosure relates to a pharmaceutical composition for preventing or treating lung disease, including a GIP derivative or a long-acting conjugate thereof.

### BACKGROUND ART

The lung is an organ mainly responsible for respiration, and lung disease occurs due to harmful substances, viruses, immune abnormalities, and the like. Lung disease causes decreased lung functions and respiratory discomfort. Accordingly, appropriate treatments are required depending on the cause of the disease.

Examples of lung-related diseases are interstitial lung disease, progressive fibrotic interstitial lung disease, idiopathic interstitial pneumonia, non-specific interstitial pneumonia, pulmonary fibrosis, interstitial pulmonary fibrosis, idiopathic pulmonary fibrosis, alveolitis, pneumonia, emphysema, bronchitis, chronic obstructive pulmonary disease (COPD), combined pulmonary fibrosis and emphysema (CPFE), asthma, and respiratory infections (e.g., coronavirus disease (COVID-19)). Since these lung diseases are related to each other and various symptoms appear while being mixed, it is known that a therapeutic agent should be carefully selected. The main pathogenesis of lung disease includes lung injury and inflammatory response, and fibrosis.

Specifically, when an inflammatory reaction occurs in the lung due to viruses, microorganisms, harmful substances, etc., secretion of inflammatory cytokines (e.g., IL-1, IL-6, TNF-α) by alveolar macrophages, attraction of neutrophils, the secretion of protease, and the like may occur, and due to the secretion of protease (e.g., elastase), the elasticity and structure of the lung tissue are impaired, resulting in fibrosis of the lung. Inflammation and fibrosis of the lung precede the progression of many lung diseases. Accordingly, they are considered as fundamental mechanisms in the prevention and treatment of lung diseases.

Chronic obstructive pulmonary disease (COPD), a typical example of lung disease, is a disease in which airway obstruction occurs gradually as the airway narrows due to an abnormal inflammatory response of the lungs by tobacco, air pollution, or toxic inhaled substances. COPD is characterized by bronchitis and emphysema. In particular, smoking is known to be the main cause of COPD. Smoking acts as a strong irritant in the lung tissue, increases the production of various proinflammatory factors, growth factors, oxidative substances, and chemotactic factors, and activates the inflammatory signal transduction system to promote migration of many inflammatory cells, including neutrophils and macrophages. Thus, lung inflammation is worsen, which in turn causes abnormal changes in lung tissues, such as airway wall thickening and pulmonary fibrosis, and thus, deteriorates lung function. Accordingly, amelioration of lung inflammation is considered as a treatment method for the prevention and treatment of COPD.

As described above, since lung inflammation and fibrosis are major causes of the onset and development of lung diseases, amelioration of lung inflammation and fibrosis has been studied as a treatment mechanism for various lung diseases.

Glucose-dependent insuliontropic polypeptide (GIP) is a representative gastrointestinal hormone and a neurohormone, and is secreted upon stimulation by food intake. GIP is a hormone composed of 42 amino acids secreted from the K cells of the small intestine. Depending on the blood glucose concentration, GIP promotes insulin secretion in the pancreas and helps to lower the blood glucose level. There are reports of the activity increase effect and anti-inflammatory effects of GLP-1. According to research, it is known that the GIP receptor is expressed in several tissues, one of which is the lung. Therefore, GIP can be expected to be effective as a therapeutic agent for lung disease through the anti-inflammatory effect thereof by acting directly on the lungs.

In addition, although various studies on COPD are being conducted so far, the development of a practical and effective therapeutic agent is still insufficient, and there is a need for continuous development of a therapeutic agent.

Accordingly, the inventors of the present application have developed a long-acting GIP derivative conjugate that exhibits high activity at the human GIP receptor and has improved persistence in the body, and confirmed the possibility of the same as a therapeutic agent for lung disease, thereby completing the present disclosure.

### DESCRIPTION OF EMBODIMENTS

### TECHNICAL PROBLEM

The present disclosure provides a pharmaceutical composition for preventing or treating lung disease, including a GIP derivative, a pharmaceutically acceptable salt thereof, a solvate thereof, or a conjugate thereof.

The present disclosure provides a method of preventing or treating lung disease, including administering an effective amount of the GIP derivative, a pharmaceutically acceptable salt thereof, a solvate thereof, or a conjugate thereof, or a pharmaceutical composition, to a subject in need thereof.

The present disclosure provides use of the GIP derivative, a pharmaceutically acceptable salt thereof, a solvate thereof, or a conjugate thereof for use in the preparation of a medicament for the prevention or treatment of lung disease.

### SOLUTION TO PROBLEM

Throughout this specification, for naturally occurring amino acids, conventional one-letter and three-letter codes are used, and for other amino acids, such as Aib (α-aminoisobutyric acid) and the like, commonly accepted three-letter codes. Also, amino acids referred to by abbreviation herein are described according to the IUPAC-IUB nomenclature.

| | |
|---|---|
| Alanine Ala, A | Arginine Arg, R |
| Asparagine Asn, N | Aspartic Acid Asp, D |

| | |
|---|---|
| Cysteine Cys, C | Glutamic acid Glu, E |
| Glutamine Gln, Q | Glycine Gly, G |
| Histidine His, H | Isoleucine Ile, I |
| Leucine Leu, L | Lysine Lys, K |
| Methionine Met, M | Phenylalanine Phe, F |
| Proline Pro, P | Serine Ser, S |
| Threonine Thr, T | Tryptophan Trp, W |
| Tyrosine Tyr, Y | Valine Val, V |

An aspect provides a pharmaceutical composition for preventing or treating lung disease, including a GIP derivative, a pharmaceutically acceptable salt thereof, or a solvate thereof.

GIP (glucose-dependent insulinotropic polypeptide or gastric inhibitory polypeptide) is a hormone secreted from K cells in the small intestine when stimulated by food intake, and was first reported as a substance involved in regulating blood glucose levels.

The "GIP derivative" may be a derivative of native GIP in which at least one amino acid is modified in the native GIP sequence. The variation may be selected from substitution, addition, deletion, modification, and combinations of two or more thereof. The added amino acid sequence may be derived from a native GIP amino acid sequence, but is not limited thereto.

The GIP derivative may be a peptide having activity with respect to a GIP receptor. The "peptide having activity with respect to a GIP receptor" has a significant level of activity with respect to the GIP receptor, and specifically, the *in vitro* activity with respect to the GIP receptor is, of the native ligand (native GIP), about 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, 100% or more, 100% to 500%, or 100% to 200%. The method of measuring the *in vitro* activity of the peptide having activity with respect to the GIP receptor may be understood by referring to Example 2, but is not limited thereto, and any method known in the art may be appropriately used to measure the *in vitro* activity.

"About" is a range inclusive of ±0.5, ±0.4, ±0.3, ±0.2, ±0.1, etc., including, but not limited to, all numerical values in a range equal to or similar to the numerical value following the term "about".

In an embodiment, the GIP derivative may be one in which 1, 2, 3, 4, 5, 6, 7, 8, 9, 10 or more amino acids are conservatively substituted in the native or unmutated GIP protein. However, the present disclosure is not limited thereto.

"Conservative substitution" refers to substituting one amino acid for another amino acid having similar structural and/or chemical properties. The GIP derivative may have, for example, one or more conservative substitutions while still retaining the biological activity of the native or unmutated GIP protein. Such amino acid substitutions may generally occur based on similarity of the residues in the polarity, charge, solubility, hydrophobicity, hydrophilicity and/or amphipathic nature. For example, positively charged (basic) amino acids include arginine, lysine, and histidine; negatively charged (acidic) amino acids include glutamic acid and aspartic acid; aromatic amino acids include phenylalanine, tryptophan and tyrosine; and hydrophobic amino acids include alanine, valine, isoleucine, leucine, methionine, phenylalanine, tyrosine, and tryptophan. In addition, amino acids can be classified into amino acids with electrically charged side chains and amino acids with uncharged side chains. Amino acids having a charged side chain include aspartic acid, glutamic acid, lysine, arginine, and histidine, and amino acids having an uncharged side chain can be sub-classified as nonpolar amino acids or polar amino acids. The nonpolar amino acids may include glycine, alanine, valine, leucine, isoleucine, methionine, and proline; and polar amino acids may include serine, threonine, cysteine, asparagine, and glutamine. Due to the conservative substitutions with amino acids having similar properties, it is expected to obtain the same or similar activity.

The GIP derivative may be non-naturally occurring.

The GIP derivative may be an isolated peptide.

In an embodiment, the GIP derivative is a peptide including an amino acid sequence represented by General Formula 1:

Tyr-Aib(aminoisobutyric acid)-G!u-G!y-Thr-Phe-!!e-Ser-Asp-Tyr-Ser-!!e-Xaa13- Xaa14-Xaa15-Xaa16-Xaa17-Ala-Xaa19-Xaa20-Xaa21 -Phe-Xaa23-Xaa24-Trp-Leu- Xaa27-Xaa28-Xaa29-Xaa30-Xaa31-Xaa32-Xaa33-Xaa34-Xaa35-Xaa36-Xaa37-Xaa38- Xaa39-Xaa40-Xaa41-Xaa42-Xaa43 (General Formula 1)

In General Formula 1,
Xaa13 is alanine (Ala, A), Aib, tyrosine (Tyr, Y), or glutamine (Gln, Q),
Xaa14 is methionine (Met, M) or leucine (Leu, L),
Xaa15 is aspartic acid (Asp, D) or glutamic acid (Glu, E),
Xaa16 is alanine (Ala, A), lysine (Lys, K), or glycine (Gly, G);
Xaa17 is isoleucine (Ile, I) or glutamine (Gln, Q),
Xaa19 is glutamine (Gln, Q) or alanine (Ala, A),
Xaa20 is glutamine (Gln, Q), Aib, or lysine (Lys, K),
Xaa21 is aspartic acid (Asp, D) or glutamic acid (Glu, E),
Xaa23 is valine (Val, V) or isoleucine (Ile, I),
Xaa24 is asparagine (Asn, N), alanine (Ala, A), or glutamine (Gln, Q);
Xaa27 is leucine (Leu, L) or isoleucine (Ile, I),
Xaa28 is alanine (Ala, A) or Aib,
Xaa29 is glutamine (Gln, Q) or glycine (Gly, G),
Xaa30 is lysine (Lys, K), glycine (Gly, G), or histidine (His, H),
Xaa31 is proline (Pro, P), glycine (Gly, G), or cysteine (Cys, C),
Xaa32 is serine (Ser, S) or lysine (Lys, K) or absent,
Xaa33 is serine (Ser, S) or lysine (Lys, K) or absent,
Xaa34 is glycine (Gly, G) or asparagine (Asn, N) or absent,
Xaa35 is alanine (Ala, A) or aspartic acid (Asp, D) or absent,
Xaa36 is proline (Pro, P) or tryptophan (Trp, W) or absent,
Xaa37 is proline (Pro, P) or lysine (Lys, K) or absent,
Xaa38 is proline (Pro, P) or histidine (His, H) or absent,
Xaa39 is serine (Ser, S), asparagine (Asn, N), or cysteine (Cys, C), or absent,
Xaa40 is cysteine (Cys, C) or isoleucine (Ile, I) or absent,
Xaa41 is threonine (Thr, T) or absent,
Xaa42 is glutamine (Gln, Q) or absent, and
Xaa43 is cysteine (Cys, C) or absent.

Examples of such peptides may include any one amino acid sequence selected from the group consisting of SEQ ID NOS: 1 to 26.

In an embodiment, the peptide may include an amino acid sequence represented by General Formula 2:

Tyr-Aib (aminoisobutyric acid)-Glu-Gly-Thr-Phe-Ile-Ser-Asp-Tyr-Ser-Ile-Xaa13- Xaa14-Xaa15-Xaa16-Xaa17-Ala-Xaa19-Xaa20-Xaa21 -Phe-Val- Xaa24-Trp-Leu-Xaa27- Xaa28-Xaa29-Xaa30-Xaa31-Xaa32-Xaa33-Xaa34-Xaa35-Xaa36-Xaa37-Xaa38-Xaa39- Xaa40-Xaa41-Xaa42-Xaa43 (General Formula 2)

In General Formula 2,
Xaa13 is alanine (Ala, A), Aib, or tyrosine (Tyr, Y),
Xaa14 is methionine (Met, M) or leucine (Leu, L),
Xaa15 is aspartic acid (Asp, D) or glutamic acid (Glu, E),
Xaa16 is alanine (Ala, A) or lysine (Lys, K),
Xaa17 is isoleucine (Ile, I) or glutamine (Gln, Q),
Xaa19 is glutamine (Gln, Q) or alanine (Ala, A),
Xaa20 is glutamine (Gln, Q), Aib, or lysine (Lys, K);
Xaa21 is aspartic acid (Asp, D) or glutamic acid (Glu, E),
Xaa24 is asparagine (Asn, N), or glutamine (Gln, Q),
Xaa27 is leucine (Leu, L) or isoleucine (Ile, I),
Xaa28 is alanine (Ala, A) or Aib,
Xaa29 is glutamine (Gln, Q) or glycine (Gly, G),
Xaa30 is lysine (Lys, K), glycine (Gly, G), or histidine (His, H);
Xaa31 is proline (Pro, P) or glycine (Gly, G),
Xaa32 is serine (Ser, S) or lysine (Lys, K),
Xaa33 is serine (Ser, S) or lysine (Lys, K),
Xaa34 is glycine (Gly, G) or asparagine (Asn, N);
Xaa35 is alanine (Ala, A) or aspartic acid (Asp, D);
Xaa36 is proline (Pro, P) or tryptophan (Trp, W),
Xaa37 is proline (Pro, P) or lysine (Lys, K),
Xaa38 is proline (Pro, P) or histidine (His, H),
Xaa39 is serine (Ser, S), asparagine (Asn, N), or cysteine (Cys, C);
Xaa40 is cysteine (Cys, C) or isoleucine (Ile, I) or absent;
Xaa41 is threonine (Thr, T) or absent,
Xaa42 is glutamine (Gln, Q) or absent, and
Xaa43 is cysteine (Cys, C) or absent.

Examples of such peptides may include any one amino acid sequence selected from the group consisting of SEQ ID NOS: 11, 17, and 19 to 26.

In an embodiment, the peptide may include an amino acid sequence represented by General Formula 3:

Tyr-Aib (aminoisobutyric acid)-Glu-Gly-Thr-Phe-Ile-Ser-Asp-Tyr-Ser-Ile-Xaa13- Xaa14-Xaa15-Xaa16-Xaa17-Ala-Xaa19-Xaa20-Xaa21-Phe-Val- Asn-Trp-Leu-Leu- Xaa28-Xaa29-Xaa30-Xaa31-Xaa32-Xaa33-Xaa34-Xaa35-Xaa36-Xaa37-Xaa38-Xaa39- Xaa40-Xaa41-Xaa42-Xaa43 (General Formula 3)

In General Formula 3,
Xaa13 is alanine (Ala, A) or Aib,
Xaa14 is methionine (Met, M) or leucine (Leu, L),
Xaa15 is aspartic acid (Asp, D) or glutamic acid (Glu, E),
Xaa16 is alanine (Ala, A) or lysine (Lys, K),
Xaa17 is isoleucine (Ile, I) or glutamine (Gln, Q),
Xaa19 is glutamine (Gln, Q) or alanine (Ala, A),
Xaa20 is glutamine (Gln, Q) or Aib,
Xaa21 is aspartic acid (Asp, D) or glutamic acid (Glu, E),
Xaa28 is alanine (Ala, A) or Aib,
Xaa29 is glutamine (Gln, Q) or glycine (Gly, G),
Xaa30 is lysine (Lys, K), glycine (Gly, G), or histidine (His, H);
Xaa31 is proline (Pro, P) or glycine (Gly, G),
Xaa32 is serine (Ser, S) or lysine (Lys, K),
Xaa33 is serine (Ser, S) or lysine (Lys, K),
Xaa34 is glycine (Gly, G) or asparagine (Asn, N);
Xaa35 is alanine (Ala, A) or aspartic acid (Asp, D);
Xaa36 is proline (Pro, P) or tryptophan (Trp, W),
Xaa37 is proline (Pro, P) or lysine (Lys, K),
Xaa38 is proline (Pro, P) or histidine (His, H),
Xaa39 is serine (Ser, S) or asparagine (Asn, N),
Xaa40 is cysteine (Cys, C) or isoleucine (Ile, I),
Xaa41 is threonine (Thr, T) or absent,
Xaa42 is glutamine (Gln, Q) or absent, and
Xaa43 is cysteine (Cys, C) or absent.

Examples of such peptides may include any one amino acid sequence selected from the group consisting of SEQ ID NOS: 11, 17, 21, and 24.

In an embodiment, in General Formula 3,
Xaa13 is alanine (Ala, A) or Aib,
Xaa14 is leucine (Leu, L),
Xaa15 is aspartic acid (Asp, D) or glutamic acid (Glu, E),
Xaa16 is lysine (Lys, K),
Xaa17 is glutamine (Gln, Q),
Xaa19 is glutamine (Gln, Q) or alanine (Ala, A),
Xaa20 is glutamine (Gln, Q) or Aib,
Xaa21 is aspartic acid (Asp, D) or glutamic acid (Glu, E),
Xaa28 is alanine (Ala, A) or Aib,
Xaa29 is glutamine (Gln, Q),
Xaa30 is glycine (Gly, G), or histidine (His, H);
Xaa31 is proline (Pro, P),
Xaa32 is serine (Ser, S),
Xaa33 is serine (Ser, S),
Xaa34 is glycine (Gly, G),
Xaa35 is alanine (Ala, A),
Xaa36 is proline (Pro, P),
Xaa37 is proline (Pro, P),
Xaa38 is proline (Pro, P),
Xaa39 is serine (Ser, S),
Xaa40 is cysteine (Cys, C), and
Xaa41 to Xaa43 may be absent.

Examples of such peptides may include any one amino acid sequence selected from the group consisting of SEQ ID NOS: 17, 21, and 24.

In an embodiment, in General Formula 1,
Xaa13 is alanine (Ala, A),
Xaa14 is methionine (Met, M),
Xaa15 is aspartic acid (Asp, D),
Xaa16 is alanine (Ala, A),
Xaa17 is isoleucine (Ile, I),
Xaa19 is glutamine (Gln, Q),
Xaa20 is glutamine (Gln, Q),
Xaa21 is aspartic acid (Asp, D),
Xaa23 is valine (Val, V),
Xaa24 is asparagine (Asn, N),
Xaa27 is leucine (Leu, L),
Xaa28 is alanine (Ala, A),
Xaa29 is glutamine (Gln, Q),
Xaa30 is lysine (Lys, K),
Xaa31 is glycine (Gly, G),
Xaa32 is lysine (Lys, K),
Xaa33 is lysine (Lys, K),
Xaa34 is asparagine (Asn, N),
Xaa35 is aspartic acid (Asp, D),
Xaa36 is tryptophan (Trp, W),
Xaa37 is lysine (Lys, K),
Xaa38 is histidine (His, H),
Xaa39 is asparagine (Asn, N),
Xaa40 is isoleucine (Ile, I),
Xaa41 is threonine (Thr, T),
Xaa42 is glutamine (Gln, Q), and
Xaa43 may be cysteine (Cys, C).

However, when any one amino acid of Xaa32 to Xaa43 in General Formulae 1 to 3 is absent, the subsequent amino acid sequence may be absent. As an example, when Xaa32 is absent, Xaa33 to Xaa43 may be absent. As another example, when Xaa41 is absent, Xaa42 to Xaa43 may be absent.

In an embodiment, the peptide may include any one amino acid sequence selected from the group consisting of SEQ ID NOS: 1 to 26. In addition, the peptide consists essentially of any one amino acid sequence selected from the group consisting of SEQ ID NOS: 1 to 26, or the peptide may consist of any one amino acid sequence selected from the group consisting of SEQ ID NOS: 1 to 26. The peptide including (or consisting essentially of, or consisting of) the amino acid sequence of any one of SEQ ID NOS: 1 to 26 may have an *in vitro* activity with respect to a GIP receptor of about 1% or more of native GIP. Accordingly, the peptides of SEQ ID NOS: 1 to 26 may exhibit higher activity than native GIP, thereby exhibiting excellent pharmacological effects.

In an embodiment, the peptide may include any one amino acid sequence selected from the group consisting of SEQ ID NOS: 1 to 7 and 10 to 26. In addition, the peptide consists essentially of any one amino acid sequence selected from the group consisting of SEQ ID NOS: 1 to 7 and 10 to 26, or the peptide may consist of any one amino acid sequence selected from the group consisting of SEQ ID NOS: 1 to 7 and 10 to 26. The peptide including (or consisting essentially of, or consisting of) the amino acid sequence of any one amino acid sequence of SEQ ID NOS: 1 to 7 and 10 to 26 may have, with respect to a GIP receptor, an *in vitro* activity of about 10% or more of native GIP. Accordingly, the peptides of SEQ ID NOS: 1 to 7 and 10 to 26 may exhibit significantly higher activity than native GIP, thereby exhibiting excellent pharmacological effects.

In an embodiment, the peptide may include any one amino acid sequence selected from the group consisting of SEQ ID NOS: 1, 11 to 13, 15, 17, and 19 to 26. In addition, the peptide consists essentially of any one amino acid sequence selected from the group consisting of SEQ ID NOS: 1, 11 to 13, 15, 17, and 19 to 26, or the peptide may consist of any one amino acid sequence selected from the group consisting of SEQ ID NOS: 1, 11 to 13, 15, 17, and 19 to 26. The peptide including (or consisting essentially of, or consisting of) the amino acid sequence of any one amino acid sequence of SEQ ID NOS: 1, 11 to 13, 15, 17, and 19 to 26 may have, with respect to a GIP receptor, an *in vitro* activity of about 30% or more of native GIP. Accordingly, the peptides of SEQ ID NOS: 1, 11 to 13, 15, 17, and 19 to 26 may exhibit significantly higher activity than native GIP, thereby exhibiting excellent pharmacological effects.

In an embodiment, the peptide may include any one amino acid sequence selected from the group consisting of SEQ ID NOS: 11, 17, and 19 to 26. In addition, the peptide consists essentially of any one amino acid sequence selected from the group consisting of SEQ ID NOS: 11, 17, and 19 to 26, or the peptide may consist of any one amino acid sequence selected from the group consisting of SEQ ID NOS: 11, 17, and 19 to 26. The peptide including (or consisting essentially of, or consisting of) the amino acid sequence of any one amino acid sequence of SEQ ID NOS: 11, 17, and 19 to 26 may have, with respect to a GIP receptor, an *in vitro* activity of about 60% or more of native GIP. Accordingly, the peptides of SEQ ID NOS: 11, 17, and 19 to 26 may exhibit significantly higher activity than native GIP, thereby exhibiting excellent pharmacological effects.

In an embodiment, the peptide may include any one amino acid sequence selected from the group consisting of SEQ ID NOS: 11, 17, 21 and 24. In addition, the peptide consists essentially of any one amino acid sequence selected from the group consisting of SEQ ID NOS: 11, 17, 21 and 24, or the peptide may consist of any one amino acid sequence selected from the group consisting of SEQ ID NOS: 11, 17, 21 and 24. The peptide including (or consisting essentially of, or consisting of) the amino acid sequence of any one amino acid sequence of SEQ ID NOS: 11, 17, 21 and 24 may have, with respect to a GIP receptor, an *in vitro* activity of about 100% or more, for example, about 110% or more, of native GIP. Accordingly, the peptides of SEQ ID NOS: 11, 17, 21 and 24 may exhibit significantly higher activity than native GIP, thereby exhibiting excellent pharmacological effects.

In an embodiment, the peptide may include any one amino acid sequence selected from the group consisting of SEQ ID NOS: 17, 21, and 24. In addition, the peptide consists essentially of any one amino acid sequence selected from the group consisting of SEQ ID NOS: 17, 21, and 24, or the peptide may consist of any one amino acid sequence selected from the group consisting of SEQ ID NOS: 17, 21, and 24. The peptide including (or consisting essentially of, or consisting of) the amino acid sequence of any one of SEQ ID NOS: 17, 21, and 24, in particular, a long-acting conjugate type of the peptides may have an in vitro activity with respect to a GIP receptor of about 120% or more of native GIP. Accordingly, the peptides of SEQ ID NOS: 17, 21, and 24, in particular, a long-acting conjugate type of the peptides may exhibit significantly higher activity than native GIP, thereby exhibiting excellent pharmacological effects.

Even when it is described herein as a "peptide consisting of a specific sequence number, as long as a peptide has the same or corresponding activity as or to a peptide consisting of the amino acid sequence set forth in a corresponding sequence number, addition of meaningless sequences before and after the amino acid sequence set forth in the corresponding sequence number or mutations that may naturally occur or silent mutations thereof are not excluded, and even when the sequence addition or mutations occur, these cases belong to the scope of the present disclosure. That is, even when there is a difference in the sequence, as long as peptides exhibit a certain level of sequence identity or more and an activity with respect to the GIP receptor, this case may fall in the scope of the present disclosure. In detail, the peptide may include an amino acid sequence having an identity of 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99%, with respect to the amino acid sequences of SEQ ID NOS: 1 to 26, but is not limited thereto.

The term "homology" or "identity" refers to the degree to which two given amino acid sequences or base sequences are related to each other and may be expressed as a percentage. Whether any two peptide sequences have homology, similarity or identity can be determined by using, for example, a known computer algorithm such as the "FASTA" program using a default parameter as disclosed in Pearson et al (1988) [Proc. Natl. Acad. Sci. USA 85]: 2444. Alternatively, the determination may be performed using the Needleman-Wunsch algorithm (Needleman and Wunsch, 1970, J. Mol. Biol. 48: 443-453), as performed in the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information Database, or ClustalW, can be used to determine homology, similarity or identity.

The homology, similarity or identity of peptides may be determined by comparing sequence information by using, for example, a GAP computer program such as Needleman et al. (1970), J Mol Biol. 48: 443, as published in Smith and Waterman, Adv. Appl. Math (1981) 2:482. In summary, the GAP program define as a value obtained by dividing the number of similarly aligned symbols (i.e., amino acids) by the total number of symbols in the shorter sequence out of two sequences. Default parameters for the GAP program may include: (1) a binary comparison matrix (containing the value of 1 for identity and the value of 0 for non-identity) and weighted comparison matrix of Gribskov et al(1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10, and a gap extension penalty of 0.5); and (3) no penalty for end gaps. Thus, the terms "homology" and "identity" used herein refer to a relevance between sequences.

In an embodiment, a peptide including the amino acid sequence of General Formula 1 according to an aspect may be prepared by a combination of various methods for preparing various peptides.

A peptide according to an aspect may be synthesized by a method well known in the art according to its length, for example, an automatic peptide synthesizer, or may be produced by a genetic engineering technique. For example, the peptide may be prepared by standard synthetic methods, recombinant expression systems, or any other method in the art. Accordingly, a peptide according to an aspect may be synthesized by a number of methods including, but not limited to, for example, methods including:
(a) a method in which a peptide is synthesized stepwise or by fragment assembly through solid or liquid phase methods, and a final peptide product is isolated and purified; or
(b) a method in which a nucleic acid construct encoding a peptide is expressed in a host cell, and the expression product is recovered from a host cell culture; or
(c) a method in which cell-free in vitro expression of a nucleic acid construct encoding a peptide is performed and the expression product is recovered; or
a method in which fragments of a peptide are obtained by any combination of (a), (b), and (c), and then the fragments are ligated to obtain a peptide, which was then recovered.

In addition, the preparation of the peptide may include: modification using L- or D-form amino acids, and/or non-natural amino acids; and/or modification of the native sequence, for example, modification of side chain functional groups, intramolecular covalent bonds (e.g., inter-side chain ring formation), methylation, acylation, ubiquitination, phosphorylation, aminohexylation, biotinylation, etc. In addition, the modification includes all substitutions with non-native compounds.

Substituted or added amino acids used for the modification may include atypical or non-naturally occurring amino acids as well as the 20 amino acids commonly found in human proteins. Commercial sources of atypical amino acids may include, but are not limited to, Sigma-Aldrich, ChemPep and Genzyme pharmaceuticals. For example, aminoisobutyric acid (Aib) may be prepared by synthesizing amino acids of Strecker in acetone, but the preparation method therefor is not limited thereto. Peptides containing such atypical or non-naturally occurring amino acids and typical peptide sequences may be synthesized and purchased from commercial peptide synthesis companies, for example, American peptide company or Bachem in the United States, or Anygen in Korea, but the synthesis and purchasing methods are not limited thereto.

In addition, the peptide may have a non-modified N-terminus and/or C-terminus. However, a case where the N-terminus and/or C-terminus thereof is chemically modified or protected with an organic group, or an amino acid is added to the end of the peptide, etc. to cause modification, which is performed to protect from proteases *in vivo* and to increase stability, is also included in the scope of the peptide according to the aspect. When the C-terminus is not modified, the terminus of the peptide has a free carboxyl group, but embodiment of the present disclosure is limited thereto.

In particular, in the case of a chemically synthesized peptide, the N- and C-termini are charged. Accordingly, the N-terminus and/or the C-terminus may be modified to remove these charges. For example, the N-terminus may undergo acetylation and/or the C-terminus may undergo, but the present disclosure is not particularly limited thereto.

In an embodiment, the peptide may have the C-terminus which is not modified or is amidated, but embodiments of the present disclosure are not limited thereto.

In an embodiment, the peptide may be an amidated C-terminus.

The peptide includes the peptide itself, a salt thereof (for example, a pharmaceutically acceptable salt of the peptide), or a solvate thereof.

The type of the salt is not particularly limited. However, the type of the salt is safe and effective for the subject, for example, mammals. However, embodiments of the present disclosure are not limited thereto.

In addition, the peptide may be in any pharmaceutically acceptable form.

The term "pharmaceutically acceptable" refers to an amount that is sufficient to exhibit a therapeutic effect and that does not cause side effects, and may be easily determined by those skilled in the art according to factors well known in the medical field, such as the type of disease, the patient's age, weight, health, sex, the patient's sensitivity to the drug, the route of administration, an administration method, the number of administration, a treatment period, drugs used for combination, or concurrently used drugs.

In an embodiment, the peptide may be in the form of a pharmaceutically acceptable salt thereof. The salt may include conventional acid addition salts used in the pharmaceutical field, for example, in the field of treating lung diseases, and examples of such acid addition salts are: salts derived from inorganic acids such as hydrochloric acid, hydrobromic acid, sulfuric acid, sulfamic acid, phosphoric acid, or nitric acid; and salts derived from organic acids such asacetic acid, propionic acid, succinic acid, glycolic acid, stearic acid, citric acid, maleic acid, malonic acid, methanesulfonic acid, tartaric acid, malic acid, phenylacetic acid, glutamic acid, benzoic acid, salicylic acid, 2-acetoxybenzoic acid, fumaric acid, toluenesulfonic acid, oxalic acid, or trifluoroacetic acid. In addition, the salt may be a base addition salt such as ammonium, dimethylamine, monomethylamine, monoethylamine, or diethylamine. The salt also includes conventional metal salt forms, for example salts derived from metals such as lithium, sodium, potassium, magnesium, or calcium. The acid addition salts, base addition salts, or metal salts may be prepared according to a conventional method. Pharmaceutically acceptable salts and general methodologies for the preparation of the same are well known in the art. For example, the document [P. Stahl, et al. Handbook of Pharmaceutical Salts: Properties, Selection and Use, 2nd Revised Edition (Wiley-VCH, 2011)]; [S.M. Berge, et al., "Pharmaceutical Salts," Journal of Pharmaceutical Sciences, Vol. 66, No. 1, January 1977] may be referred to.

For the condensation of a protected amino acid or peptide, various activating reagents useful for peptide synthesis, for example, trisphosphonium salt, tetramethyluronium salt, carbodiimide, and the like may be used. Examples of trisphosphonium salts include benzotriazol-1-yloxytris(pyrrolagino) phosphonium hexafluorophosphate (PyBOP), bromotris(pyrrolazino)phosphonium hexafluorophosphate (PyBroP), 7-azabenzotriazol-1-yloxytris(pyrrolazino)phosphonium hexafluorophosphate (PyAOP), examples of tetramethyluronium salts are 2-(1H-benzotriazol-1-yl)-1,1,3,3-tetramethyluronium hexafluorophosphate (HBTU), 2-(7-azabenzotriazol-1-yl)-1,1,3,3-tetramethyluronium hexafluoro phosphate (HATU), 2-(1H-benzotriazol-1-yl)-1,1,3,3-tetramethyluroniumtetrafluoroborate (TBTU), 2-(5-norbornane-2,3-dicarboxyimide)-1,1,3,3-tetramethyluronium tetrafluoroborate (TNTU), O-(N-succimidyl)-1,1,3,3-tetramethyluronium tetrafluoroborate (TSTU), and examples of carbodiimides include N,N'-dicyclohexylcarbodiimide (DCC), N,N'-diisopropylcarbodiimide (DIPCDI), and N-ethyl-N '-(3-dimethylaminopropyl)carbodiimide hydrochloride (EDCl·HCl). For condensation using these salts, racemization inhibitors [e.g., N-hydroxy-5-norbornene-2,3-dicarboxylic acid imide (HONB), 1-hydroxybenzotriazole (HOBt), 1-hydroxy-7-azabenzotriazole (HOAt), 3,4-dihydro-3-hydroxy-4-oxo-1,2,3-benzotriazine (HOOBt), ethyl 2-cyano-2-(hydroxyl mino)acetate (Oxyma), and the like] may be added. The solvent used for the condensation may be appropriately selected from those known to be useful for the peptide condensation reaction. For example, acid amides such as anhydrous or water-containing N,N-dimethylformamide, N,N-dimethylacetamide, N-methylpyrrolidone, etc.; halogenated hydrocarbons such as methylene chloride, chloroform, etc.; alcohols such as trifluoroethanol and phenol; sulfoxides such as dimethylsulfoxide; tertiary amines such as pyridine; ethers such as dioxane and tetrahydrofuran; nitriles such as acetonitrile and propionitrile; esters, such as methyl acetate and ethyl acetates; or suitable mixtures thereof, may be used. The reaction temperature is appropriately selected from a range known to be usable for a peptide binding reaction, and is usually selected from the range of about -20 °C to 90 °C. Activated amino acid derivatives may be usually used in 1.5 to 6-fold excess. In solid-phase synthesis, when the test using the ninhydrin reaction indicates that the condensation is insufficient, sufficient condensation may be carried out by repeating the condensation reaction without removing the protecting group. When the condensation is still insufficient after repeating the reaction, the unreacted amino acid may be acetylated with an acid anhydride, acetylimidazole, or the like, so that the influence on the subsequent reaction may be avoided.

Examples of protecting groups for the amino group of the starting amino acid are benzyloxycarbonyl (Z), tert-butoxycarbonyl (Boc), tert-pentyloxycarbonyl, isobornyloxycarbonyl, 4-methoxybenzyloxycarbonyl, 2-chlorobenzyloxycarbonyl (CI-Z), 2-bromobenzyloxycarbonyl (Br-Z), adamantyloxycarbonyl, trifluoroacetyl, phthaloyl, formyl, 2-nitro phenylsulfenyl, diphenylphosphinothioyl, 9-fluorenylmethyloxycarbonyl (Fmoc), trityl, and the like.

Examples of a carboxyl-protecting group for the starting amino acid include, in addition to the C₁₋₆ alkyl group, the C₃₋₁₀ cycloalkyl group, and the C₇₋₁₄ aralkyl group, aryl, 2-adamantyl, 4-nitrobenzyl, 4-methoxy benzyl, 4-chlorobenzyl, phenacyl and benzyloxycarbonylhydrazide, tert-butoxycarbonylhydrazide, tritylhydrazide, and the like.

The hydroxyl group of serine or threonine may be protected by, for example, esterification or etherification. Examples of groups suitable for esterification include lower (C₂₋₄) alkanoyl groups such as acetyl groups, aroyl groups such as benzoyl groups, and groups derived from organic acids and the like. Also, examples of suitable groups for etherification include benzyl, tetrahydropyranyl, tert-butyl (Bu^{t}), trityl (Trt), and the like.

Examples of the protecting group for the phenolic hydroxyl group of tyrosine include Bzl, 2,6-dichlorobenzyl, 2-nitrobenzyl, Br-Z, tert-butyl, and the like.

Examples of the protecting group for imidazole of histidine include p-toluenesulfonyl (Tos), 4-methoxy-2,3,6-trimethylbenzenesulfonyl (Mtr), dinitrophenyl (DNP), benzyloxymethyl (Bom), tert-butoxymethyl (Bum), Boc, Trt, Fmoc, and the like.

Examples of the protecting group for the guanidino group of arginine include Tos, Z, 4-methoxy-2,3,6-trimethylbenzenesulfonyl (Mtr), p-methoxybenzenesulfonyl (MBS), 2,2, 5,7,8-pentamethylchroman-6-sulfonyl (Pmc), mesitylene-2-sulfonyl (Mts), 2,2,4,6,7-pentamethyldihydrobenzofuran-5-sulfonyl phonyl (Pbf), Boc, Z, NO₂, and the like.

Examples of the protecting group for the side chain amino group of lysine include Z, C!-Z, trifluoroacetyl, Boc, Fmoc, Trt, Mtr, 4,4-dimethyl-2,6-dioxocyclohexylidenyl (Dde), etc.

Examples of the protecting group for indolyl of tryptophan include formyl (For), Z, Boc, Mts, Mtr, and the like.

Examples of the protecting group for asparagine and glutamine include Trt, xantyl (Xan), 4,4'-dimethoxybenzhydryl (Mbh), 2,4,6-trimethoxybenzyl (Tmob), and the like.

Examples of the activated carboxyl group in the starting material include corresponding acid anhydrides, azides, and active esters [esters with alcohols (e.g. pentachlorophenol, 2,4,5-trichlorophenol, 2,4-dinitrophenol, cyanomethyl alcohol, paranitrophenol, HONB, N-hydroxysucciimide, 1-hydroxybenzotriazole (HOBt), and 1-hydroxy-7-azabenzotriazole (HOAt))]. Examples of activated amino groups in the starting material include the corresponding phosphorus amides.

Examples of methods for removing protecting groups include: a catalytic reduction in a hydrogen stream in the presence of a catalyst such as Pd-black or Pd-carbon; an acid treatment using a solution of anhydrous hydrogen fluoride, methanesulfonic acid, trifluoromethanesulfonic acid, trifluoroacetic acid (TFA), trimethylsilyl bromide (TMSBr), trimethylsilyl trifluoromethanesulfonate, tetrafluoroboric acid, tris(trisfluoro)boric acid, boron tribromide, or a mixture thereof; a base treatment using diisopropylethylamine, triethylamine, piperidine, piperazine, or the like; and reduction using sodium in liquid ammonia. The removal reaction by acid treatment described above is generally carried out at a temperature of -20 °C to 40 °C; and acid treatments may be efficiently performed by adding a cation scavenger such as anisole, phenol, thioanisole, methacresol and paracresol, dimethylsulfide, 1,4-butanedithiol, 1,2-ethanedithiol, triisopropylsilane, and the like. Further, the 2,4-dinitrophenyl group used as the protecting group for imidazole of histidine is removed by thiophenol treatment; and the formyl group used as the protecting group for the indole of tryptophan is removed by deprotection through not only acid treatment in the presence of 1,2-ethanedithiol, 1,4-butanedithiol, etc., but also alkali treatment with diluted sodium hydroxide, diluted ammonia, etc.

Protection of a functional group that should not be involved in the reaction between the starting material and the protecting group, removal of the protecting group, activation of the functional group involved in the reaction, and the like, may be appropriately selected from known protecting groups and known methods.

In regard to the peptide described herein, according to the conventional peptide marking, the left end is the N-terminus(amino terminus) and the right end is the C-terminus (carboxyl terminus). The C-terminus of the peptide may be any one of an amide (-CONH₂), a carboxyl group (-COOH), a carboxylate (-COO-), an alkylamide (-CONHR', where R' is alkyl), and an ester (-COOR', wherein R' is alkyl or aryl).

Regarding the method for producing an amide of a peptide, this may be formed by solid-phase synthesis using a resin for amide synthesis, or the α-carboxyl group of a carboxy-terminal amino acid is amidated, the peptide chain is extended to the desired chain length toward the amino group, and then, a peptide in which only the protecting group for the N-terminal α-amino group of the peptide chain is removed and a peptide in which only the protecting group for the C-terminal carboxyl group is removed from the peptide chain, are prepared, and these two peptides are condensed in a mixed solvent described above. As for the details of the condensation reaction, the same described above is applied herein. After the protected peptide obtained by condensation is purified, all protecting groups may be removed by the method described above to obtain the desired peptide. By purifying this peptide using various publicly known methods of purification of the main fraction and freeze-drying, the target amide of the peptide may be prepared.

In an embodiment, the peptide may be in the form of a solvate thereof. "Solvate" refers to a complex formed by the peptide or a salt thereof together with a solvent molecule.

In an embodiment, the peptide may be in the form of a long-acting conjugate. The conjugate may exhibit increased efficacy preservation compared to native GIP or GIP derivative, which exhibit the same or higher activity with respect to GIP receptors than native GIP, and at the same time, which is not linked to a carrier (or biocompatible material). Therefore, the conjugate may be a long-acting conjugate. The term "long-acting conjugate" refers to a conjugate with increased durability of efficacy compared to a natural GIP or a GIP derivative, to which a biocompatible material is not linked. Accordingly, the conjugate may be referred to as a "long-acting GIP derivative conjugate" or a "long-acting GIP derivative" or "long-acting GIP conjugate". Such a conjugate includes not only the above-described form, but also any form that is encapsulated in biodegradable nanoparticles.

The conjugate may be a separate conjugate.

The combination may be non-naturally occurring.

In an embodiment, the peptide may be in the form of a conjugate to which a biocompatible material that increases the half-life *in vivo* is bound. Accordingly, the pharmaceutical composition may include a conjugate in which the GIP derivative is bonded to a biocompatible material that increases the *in vivo* half-life. The biocompatible material may be mixed with a carrier.

The biocompatible material may be bonded to the GIP through a covalent chemical bond or a non-covalent chemical bond, and via a linker (L) through a covalent chemical bond, a non-covalent chemical bond, or a combination thereof. One or more amino acid side chains in the GIP derivative may be conjugated to such biocompatible materials to increase solubility and/or half-life and/or increase bioavailability *in vivo.* Such modification may also reduce clearance of therapeutic proteins and peptides. The biocompatible material described above may be water-soluble (amphiphilic or hydrophilic) and/or non-toxic and/or pharmaceutically acceptable.

The biocompatible material may be selected from a high-molecular weight polymer, fatty acid, cholesterol, albumin and fragments thereof, albumin binding material, a polymer of a repeating unit of a specific amino acid sequence, an antibody, an antibody fragment, a FcRn binding material, a connective tissue *in vivo,* nucleotide, fibronectin, transferrin, saccharide, heparin, and elastin, but embodiments of the present disclosure are not limited thereto.

The high-molecular polymer may be selected from polyethylene glycol (PEG), polypropylene glycol, an ethylene glycol-propylene glycol copolymer, polyoxyethylated polyol, polyvinyl alcohol, polysaccharide, polyvinyl ethyl ether, biodegradable polymer, lipid polymer, chitin, hyaluronic acid, oligonucleotides, and combinations thereof, and the polysaccharide may include dextran, but embodiments of the present disclosure are not limited thereto.

The polyethylene glycol is a term encompassing all forms of ethylene glycol homopolymer, a PEG copolymer, or a monomethyl-substituted PEG polymer (mPEG), but is not particularly limited thereto.

The fatty acid may have a binding force with albumin *in vivo,* but embodiments of the present disclosure are not limited thereto.

The biocompatible materials include, but are not limited to, poly-amino acids such as poly-lysine, poly-aspartic acid, and poly-glutamic acid.

In the case of the elastin, the elastin may be human tropoelastin, which is a water-soluble precursor, and may be a polymer of some sequences or some repeating units of these, for example, elastin-like polypeptides, but is not particularly limited thereto.

In an embodiment, the biocompatible material may be an FcRn binding material. Specifically, the FcRn-binding material may be an immunoglobulin Fc region, or an IgG Fc region, or an aglycosylated IgG4 Fc region, but embodiments of the present disclosure are not limited thereto.

The "Immunoglobulin Fc region" refers to a region including a heavy chain constant region 2(CH2) and/or heavy chain constant region 3(CH3) region, excluding the heavy and light chain variable regions of an immunoglobulin. The immunoglobulin Fc region may be a component constituting a moiety of the conjugate according to an aspect.

The immunoglobulin Fc region may include a hinge region in the heavy chain constant region, but embodiments of the present disclosure are not limited thereto.

In an embodiment, the immunoglobulin Fc region may include a specific hinge sequence at the N-terminus.

The term "hinge sequence" refers to a site located on a heavy chain to form a dimer of an immunoglobulin Fc fragment through an inter disulfide bond.

In an embodiment, the hinge sequence may be a modified sequence in which part of the hinge sequence having the following amino acid sequence is deleted and thus there is only one cysteine residue in the sequence, but is not limited thereto:
Glu-Ser-Lys-Tyr-Gly-Pro-Pro-Cys-Pro-Ser-Cys-Pro (SEQ ID NO: 27).

The hinge sequence may include only one cysteine residue by deleting the 8^{th} or 11^{th} cysteine residue in the hinge sequence of SEQ ID NO: 27. The hinge sequence according to an embodiment may consist of 3 to 12 amino acids, including only one cysteine residue, but is not limited thereto. In an embodiment, the hinge sequence according to an embodiment may have the following sequences: Glu-Ser-Lys-Tyr-Gly-Pro-Pro-Pro-Ser-Cys-Pro(SEQ ID NO: 28), Glu-Ser-Lys-Tyr-Gly-Pro-Pro-Cys-Pro-Ser-Pro(SEQ ID NO: 29), Glu-Ser-Lys-Tyr-Gly-Pro-Pro-Cys-Pro-Ser(SEQ ID NO: 30), Glu-Ser-Lys-Tyr-Gly-Pro-Pro-Cys-Pro-Pro(SEQ ID NO: 31), Lys-Tyr-Gly-Pro-Pro-Cys-Pro-Ser(SEQ ID NO: 32), Glu-Ser-Lys-Tyr-Gly-Pro-Pro-Cys(SEQ ID NO: 33), Glu-Lys-Tyr-Gly-Pro-Pro-Cys(SEQ ID NO: 34), Glu-Ser-Pro-Ser-Cys-Pro(SEQ ID NO: 35), Glu-Pro-Ser-Cys-Pro(SEQ ID NO: 36), Pro-Ser-Cys-Pro(SEQ ID NO: 37), Glu-Ser-Lys-Tyr-Gly-Pro-Pro-Ser-Cys-Pro(SEQ ID NO: 38), Lys-Tyr-Gly-Pro-Pro-Pro-Ser-Cys-Pro(SEQ ID NO: 39), Glu-Ser-Lys-Tyr-Gly-Pro-Ser-Cys-Pro(SEQ ID NO: 40), Glu-Ser-Lys-Tyr-Gly-Pro-Pro-Cys(SEQ ID NO: 41), Lys-Tyr-Gly-Pro-Pro-Cys-Pro(SEQ ID NO: 42), Glu-Ser-Lys-Pro-Ser-Cys-Pro(SEQ ID NO: 43), Glu-Ser-Pro-Ser-Cys-Pro(SEQ ID NO: 44), Glu-Pro-Ser-Cys(SEQ ID NO: 45), and Ser-Cys-Pro(SEQ ID NO: 46).

In an embodiment, the hinge sequence may include the amino acid sequence of SEQ ID NO: 37 (Pro-Ser-Cys-Pro) or SEQ ID NO: 46 (Ser-Cys-Pro), but is not limited thereto.

The immunoglobulin Fc region according to an embodiment may be a form in which two molecules of an immunoglobulin Fc chain form a dimer due to the presence of a hinge sequence. In addition, the conjugate of Formula 1 according to an embodiment may be a form in which one end of the linker is linked to one chain of the immunoglobulin Fc region of the dimer, but is not limited thereto.

The term "N-terminus" refers to the amino terminus of a protein or polypeptide, and may be an end of the amino terminus, or may include 1, 2, 3, 4, 5, 6, 7, 8, 9, or 10 amino acids from the end. The immunoglobulin Fc fragment of the present disclosure may include a hinge sequence at the N-terminus, but embodiments of the present disclosure are not limited thereto.

In addition, as long as the immunoglobulin Fc region has substantially the same or improved effect as the native type, the immunoglobulin Fc region may be part or all of the heavy chain constant region 1 (CH1) and/or the light chain constant region 1 (CL1) except for only the heavy and light chain variable regions of the immunoglobulin. Also, the immunoglobulin Fc region may be a region in which an amino acid sequence corresponding to CH2 and/or CH3 have been removed.

For example, the immunoglobulin Fc region may be selected from (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination of one or more domains of a CH1 domain, a CH2 domain, a CH3 domain, and a CH4 domain with an immunoglobulin hinge region or a portion of the immunoglobulin hinge region; and (f) a dimer of each domain of the heavy chain constant region and the light chain constant region, but is not limited thereto.

The immunoglobulin Fc region may be in a dimeric form, and one molecule of the GIP derivative may be covalently linked to one Fc region in the dimeric form, wherein the immunoglobulin Fc and the GIP derivative may be linked to each other by a non-peptidic polymer. In an embodiment, two molecules of the GIP derivative may be symmetrically bonded to one Fc region in a dimeric form. In this case, the immunoglobulin Fc and the GIP derivative may be linked to each other by a non-peptidic linker. However, it is not limited to the examples described above.

In addition, the immunoglobulin Fc region contains, in addition to a native amino acid sequence, a sequence derivative thereof. An amino acid sequence derivative refers to a case in which one or more amino acid residues in a natural amino acid sequence undergo deletion, insertion, non-conservative or conservative substitution, or a combination thereof so as to have a different sequence.

For example, in the case of IgG Fc, amino acid residues 214 to 238, 297 to 299, 318 to 322, or 327 to 331, known to be important for binding, may be used as suitable sites for modification. In addition, various types of derivatives may be formed in such a manner that a site capable of forming a disulfide bond is removed, some amino acids at the N-terminus of native Fc are removed, or a methionine residue may be added to the N-terminus of native Fc. In addition, in order to eliminate the effector function, the complement binding site, for example, the C1q binding site, may be removed, or the antibody dependent cell mediated cytotoxicity (ADCC) site may be removed. Techniques for preparing these sequence derivative of immunoglobulin Fc region are disclosed in International Patent Publication No. WO 97/34631 and International Patent Publication No. 96/32478.

Amino acid exchanges in proteins and peptides that do not entirely alter the activity of the molecule are known in the art (H.Neurath, R.L.Hill, The Proteins, Academic Press, New York, 1979). The most common exchanges are an exchange between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, or Asp/Gly. In some cases, modification may be made by phosphorylation, sulfation, acrylation, glycosylation, methylation, farnesylation, acetylation, and amidation.

The Fc derivative may exhibit the same biological activity as that of the Fc region and the Fc region thereof may have increased structural stability against heat, pH, etc.

In addition, the Fc region may be obtained from a native type isolated *in vivo* from animals such as humans, cows, goats, pigs, mice, rabbits, hamsters, rats, or guinea pigs, or may be a recombinant or a derivative thereof obtained from transformed animal cells or microorganisms. Herein, the method of obtaining from the native type may be a method in which whole immunoglobulin is obtained from a living body of a human or an animal and then treated with a protease. In the case of treating papain, Fab and Fc are used for cleavage, and in the case of treating pepsin, pF'c and F(ab)₂ are used for cleavage. Fc or pF'c may be separated using size-exclusion chromatography or the like. An embodiment thereof may be a recombinant immunoglobulin Fc region of which the human-derived Fc region is obtained from a microorganism.

In addition, the immunoglobulin Fc region may have a native sugar chain, an increased sugar chain compared to the native type, or a decreased sugar chain compared to the native type, or of which the sugar chain is removed. For the increase or decrease or removal of such immunoglobulin Fc sugar chains, methods of the related art such as chemical methods, enzymatic methods, and genetic engineering methods using microorganisms may be used. Herein, the immunoglobulin Fc region which is deglycosylated in the Fc, has a significantly reduced binding force with respect to complement (c1q), and may have reduced or eliminated antibody-dependent cytotoxicity or complement-dependent cytotoxicity. Accordingly, unnecessary immune responses may not be derived *in vivo.* In this respect, a form more suitable for the original purpose as a drug carrier may be an immunoglobulin Fc region which is deglycosylated or aglycosylated.

"Deglycosylation" refers to removal of glycans from an Fc region by an enzyme, and aglycosylation refers to an Fc region that is produced in a prokaryote, for example, E. coli, and is not glycosylated.

In addition, the immunoglobulin Fc region may be an Fc region derived from IgG, IgA, IgD, IgE, or IgM, or a combination or hybrid thereof. In an embodiment, the immunoglobulin Fc region is derived from IgG or IgM, which is the most abundant in human blood. In an embodiment, the immunoglobulin Fc region is derived from IgG, which is known to enhance the half-life of ligand binding proteins. In an embodiment, the immunoglobulin Fc region is an IgG4 Fc region. In an embodiment, the immunoglobulin Fc region is an aglycosylated Fc region derived from human IgG4. However, embodiments of the present disclosure are not limited thereto.

The "combination" refers to a case in which, when dimers or multimers are formed, polypeptides encoding single-chain immunoglobulin Fc regions of the same origin form bonds with single-chain polypeptides of different origins. That is, it is possible to prepare a dimer or multimer from two or more fragments selected from IgG Fc, IgA Fc, IgM Fc, IgD Fc, and Fc fragment of IgE.

The GIP derivative may be linked to a biocompatible material through a linker.

The linker may be a peptidic linker or a non-peptidic linker.

When the linker is a peptidic linker, the linker may include one or more amino acids, for example, 1 to 1000 amino acids, but is not particularly limited thereto. The peptidic linker may include Gly, Asn, and Ser residues, and may include neutral amino acids such as Thr and Ala. In order to link the biocompatible material and the GIP derivative, various known peptide linkers may be used. The copy number "n" may also be adjusted to achieve proper separation between functional moieties or to allow for optimization of the linker to maintain the necessary inter-moiety interactions. Other flexible linkers are known in the art. For example, in addition to the addition of polar amino acid residues to improve water solubility, amino acid residues such as T and A can added to maintain flexibility. The latter case includes a G linker and an S linker. Accordingly, in an embodiment, the linker may be a flexible linker including G, S, and/or T residues. The linker may have a general formula selected from (GpSs)n and (SpGs)n, where, independently, p is an integer from 1 to 10, s is 0 or is an integer from 0 to 10, the sum of p and s is an integer of 20 or less, and n is an integer from 1 to 20. Examples of the linker are (GGGGS)n, (SGGGG)n, (SRSSG)n, (SGSSC)n, (GKSSGSGSESKS)n, (RPPPPC)n, (SSPPPPC)n, (GSTSGSGKSSEGKG)n, (GSTSGSGKSSEGSGSTKG)n, (GSTSGSGKPGSGEGSTKG)n, or (EGKSSGSGSESKEF)n, and n may be an integer from 1 to 20, or from 1 to 10.

The "non-peptidic linker" includes a biocompatible polymer to which two or more repeating units are linked. The repeating units are linked to each other through any covalent bond other than a peptide bond. The non-peptidic linker may be one component constituting a moiety of the conjugate.

The "non-peptidic linker" may be used interchangeably with "non-peptidic polymer".

In an embodiment, in the conjugate, a biocompatible material and a GIP derivative are covalently bonded to each other through a non-peptidic linker having, at both ends thereof, a reactive group capable of binding to the biocompatible material, for example, an immunoglobulin Fc region, and the GIP derivative.

For example, the non-peptidic linker may be selected from fatty acids, saccharides, high-molecular weight polymers, low-molecular weight compounds, nucleotides, and combinations thereof.

Although not particularly limited thereto, the non-peptidic linker may be selected from polyethylene glycol, polypropylene glycol, ethylene glycol-propylene glycol copolymer, polyoxyethylated polyol, polyvinyl alcohol, polysaccharide, polyvinyl ethyl ether, biodegradable polymers such as polylactic acid (PLA) and polylactic-glycolic acid (PLGA), lipid polymers, chitins, hyaluronic acid, oligonucleotides, and combinations thereof. The polysaccharide may be dextran, but is not limited thereto.

In an embodiment, the non-peptidic polymer may be, but is not limited to, polyethylene glycol. Accordingly, the linker may contain an ethylene glycol repeating unit. In addition, derivatives which are already known in the art and derivatives which can be easily prepared at the level of skill in the art, are also included in the scope of the present disclosure.

The non-peptidic linker may be any polymer that is resistant to proteolytic enzymes *in vivo.* The formula weight of the non-peptidic polymer may be in the range of 1 kDa to 1000 kDa, 1 kDa to 100 kDa, or 1 kDa to 20 kDa. However, embodiments of the present disclosure are not limited thereto. In addition, as the non-peptidic linker, not only one type of polymer, but also a combination of different types of polymers may be used. In an embodiment, the formula weight of the ethylene glycol repeating unit moiety may be in the range of 1 kDa to 100 kDa, or, in the range of 1 kDa to 20 kDa.

In an embodiment, both ends of the non-peptidic linker may be bound to a biocompatible material, such as an amine group or a thiol group of an immunoglobulin Fc region, and an amine group or a thiol group of a GIP derivative, respectively.

In an embodiment, the non-peptidic polymer may have, at both termini, a reactive group capable of binding to a biocompatible material (e.g., an immunoglobulin Fc region) and a reactive group capable of binding to a GIP derivative, respectively, for example, a reactive group capable of binding to an amine group located at the N-terminus or lysine, or a thiol group of cysteine of a GIP derivative or a biocompatible material (e.g., an immunoglobulin Fc region).

In addition, the reactive group of the non-peptidic polymer, which is capable of binding to a biocompatible material, for example, an immunoglobulin Fc region and a GIP derivative, may be selected from an aldehyde group, a maleimide group, and a succinimide derivative, but embodiments of the present disclosure are not limited thereto. In this regard, an example of the aldehyde group is a propionaldehyde group or a butyl aldehyde group, but is not limited thereto. In this regard, an example of the succinimide derivative is succinimidyl valerate, succinimidyl methylbutanoate, succinimidyl methylpropionate, succinimidyl butanoate, succinimidyl propionate, N-hydroxysuccinimid, hydroxy succinimidyl, succinimidyl carboxymethyl, or succinimidyl carbonate, but is not limited thereto.

In addition, the final product resulting from reductive alkylation with aldehyde bonds is much more stable than those linked with amide bonds. The aldehyde reactive group selectively reacts with the N-terminus at a low pH, and may form a covalent bond with a lysine residue at a high pH, for example, pH 9.0.

In addition, the reactive groups at both ends of the non-peptidic linker may be the same or different from each other. For example, the non-peptidic linker may have a maleimide group at one end and an aldehyde group, a propionaldehyde group, or a butyl aldehyde group at the other end. However, as long as a biocompatible material, specifically, an immunoglobulin Fc region and a GIP derivative can be bound to each end of the non-peptidic linker, embodiments of the present disclosure are not limited thereto. For example, the non-peptidic linker may include, as a reactive group, a maleimide group at one end and an aldehyde group, a propionaldehyde group, or a butyl aldehyde group at the other end.

In the case where polyethylene glycol having a hydroxyl reactive group at both ends is used as a non-peptidic polymer, the hydroxyl group may be activated using the various reactive groups by a known chemical reaction, or a commercially available polyethylene glycol having a modified reactive group is used, to prepare a long-acting conjugate.

In an embodiment, the non-peptidic polymer may be linked to a cysteine residue of the GIP derivative, for example, the -SH group of cysteine, but embodiments of the present disclosure are not limited thereto.

When maleimide-PEG-aldehyde is used, the maleimide group may be linked to the -SH group of the GIP derivative via a thioether bond, and the aldehyde group may be linked to a biocompatible material, for example, the -NH₂ group of immunoglobulin Fc via reductive alkylation. However, embodiments are not limited thereto, and this embodiment is only an example.

In addition, in the conjugate, the reactive group of the non-peptidic polymer may be linked to -NH₂ located at the N-terminus of the immunoglobulin Fc region, but the present embodiment is only an example.

Accordingly, the conjugate according to the one aspect may be represented by Formula 1:

[Formula 1] X - L - F

where X is a GIP derivative,
L is a linker,
F is a biocompatible material that increases the *in vivo* half-life of X, and
   - represents a bond linkage between X and L and between L and F.

In Formula 1, the GIP derivative, the linker, and the biocompatible material are the same as described above.

L in Formula 1 may be La, wherein a is 0 or a natural number, provided that when a is 2 or more, each L may be independent of each other.

In an embodiment, the linker may be polyethylene glycol (PEG) represented by Formula 2, but is not limited thereto:

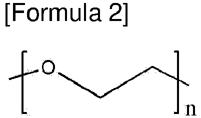

where n=10 to 2400, n=10 to 480, or n=50 to 250, but embodiments of the present disclosure are not limited thereto.

The PEG moiety in the long-acting conjugate may include, but is not limited to, in addition to the -(CH₂CH₂O)ₙ- structure, an oxygen atom intervening between a linking element and the -(CH₂CH₂O)ₙ-.

The polyethylene glycol is a term encompassing all forms of ethylene glycol homopolymer, PEG copolymer, or monomethyl-substituted PEG polymer (mPEG), but is not particularly limited thereto.

In an embodiment, "-" may represent a covalent linkage between X and L and between L and F.

It was confirmed that the GIP derivative or the conjugate thereof acts on macrophages to inhibit inflammation-related cytokines IL-6, IL-12/23 p40, IL-1β, and TNF-α, thereby exhibiting an anti-inflammatory effect, to inhibit inflammation-related genes IL-6, IL-12, and TNF-α in alveolar epithelial cells, thereby exhibiting anti-inflammatory effects, and to inhibit alveolar epithelial cell epithelial-mesenchymal transition (EMT), thereby ameliorating pulmonary fibrosis. In addition, it was confirmed that even *in vivo,* the long-acting GIP derivative ameliorates lung inflammation and emphysema.

Specifically, the pharmaceutical composition may have one or more of the following effects:
(i) inhibition of lung inflammation;
(ii) inhibition of epithelial-mesenchymal transition (EMT) of alveolar epithelial cells; and
(iii) amelioration of emphysema.

The inhibition of lung inflammation may be performed by inhibiting any one or more of the inflammation-related cytokines IL-6, IL-12/23 p40, IL-1β, and TNF-α. The inhibition of lung inflammation may be performed by inhibiting any one or more of the inflammation-related genes IL-6, IL-12, and TNF-α in alveolar epithelial cells.

In addition, the pharmaceutical composition may improve pulmonary fibrosis by inhibiting epithelial-mesenchymal transition (EMT) of alveolar epithelial cells.

Accordingly, the GIP derivative or a conjugate thereof may be used for the prevention or treatment of lung diseases, for example, the prevention or treatment of lung diseases related to lung inflammation, pulmonary fibrosis, and emphysema.

The term "prevention" refers to any action that suppresses or delays the onset of lung disease by administration of the composition.

The term "treatment" refers to any action in which the symptoms of lung disease are ameliorated or beneficial by administration of the composition.

The "lung diseases" refers to diseases occurring in the lungs, bronchial tubes, or a combination thereof. In an embodiment, the lung disease may be a disease having a pathogenesis of inflammation of the lungs, pulmonary fibrosis, emphysema, or a combination of two or more thereof. For example, the lung disease may be one or more selected from interstitial lung disease, progressive fibrotic interstitial lung disease, idiopathic interstitial pneumonia, non-specific interstitial pneumonia, pulmonary fibrosis, interstitial pulmonary fibrosis, idiopathic pulmonary fibrosis, alveolitis, pneumonia, emphysema, bronchitis, asthma, combined pulmonary fibrosis and emphysema (CPFE), chronic obstructive pulmonary disease (COPD), and coronavirus disease-19 (COVID-19), but embodiments of the present disclosure are not limited thereto.

The "interstitial lung disease (ILD)" is a generic term for diseases that show abnormal collagen deposition, accompanied by, together with proliferation of the interstitial part of the lung, infiltration of various inflammatory cells and sometimes fibrosis. The interstitial lung disease includes progressive fibrotic interstitial lung disease.

The "idiopathic interstitial pneumonia (IIP)" is a disease in which the alveolar wall becomes inflamed and fibrous, resulting in dyspnea.

The "non-specific interstitial pneumonia (NSIP)" is a type of idiopathic interstitial pneumonia, and refers to interstitial pneumonia, of patients having idiopathic interstitial pneumonia, that pathologicaly commonly cannot be classified into any of the following types: usual interstitial pneumonia (UIP), desquamative interstitial pneumonia (DIP), cryptogenic organizing pneumonia (COP), and acute interstitial pneumonia (AIP).

The "pulmonary fibrosis" is a disease in which the lung does not function normally because the lung tissue is damaged and wounded to become thick and hard. The pulmonary fibrosis may include interstitial pulmonary fibrosis, idiopathic pulmonary fibrosis, and the like.

The "alveolitis" refers to inflammation occurring in the alveoli.

The "pneumonia" refers to an inflammatory respiratory disease occurring in the lung parenchyma consisting of peripheral bronchi and alveoli.

The "emphysema" refers to a condition in which the peripheral airway alveoli are destroyed and irregularly expanded. The emphysema is one cause of chronic obstructive pulmonary disease. The most significant risk factor among the causes of emphysema is smoking, and occupational dust, chemicals, and indoor and outdoor air pollution are also related.

The "bronchitis" is an inflammation that occurs in the bronchi of the lung. The bronchitis is classified into acute bronchitis and chronic bronchitis. In an embodiment, the bronchitis may be chronic bronchitis.

The "asthma" is a disease in which symptoms such as cough and shortness of breath repeatedly occur due to severe narrowing of the bronchi due to inflammation of the bronchi when exposed to a specific causative agent.

The "combined pulmonary fibrosis and emphysema (CPFE)" is a disease that pulmonary fibrosis and emphysema develop at the same time.

The "chronic obstructive pulmonary disease (COPD)" is a disease in which airway obstruction occurs gradually as the airway narrows due to an abnormal inflammatory response of the lungs by tobacco, air pollution, or toxic inhaled substances. COPD is characterized by bronchitis and emphysema.

The "coronavirus disease" is an infectious disease transmitted by a coronavirus. The coronavirus disease may be COVID-19, but is not limited thereto. The "coronavirus disease-19 (COVID-19)" is an infectious disease infected by severe acute respiratory syndrome coronavirus 2 (SARS-CoV-2). COVID-19 may be transmitted when droplets (saliva) from an infected person penetrate the respiratory tract or the mucous membranes of the eyes, nose, or mouth. Symptoms of COVID-19 may include fever, malaise, cough, shortness of breath, pneumonia, phlegm, sore throat, headache, hemoptysis and nausea, or diarrhea. In an embodiment, the lung disease may be pneumonia (inflammation of the lungs) or pulmonary fibrosis caused by COVID-19.

The pharmaceutical composition may further include a pharmaceutically acceptable carrier. Pharmaceutically acceptable carriers may include: for oral administration, binders, lubricants, disintegrants, excipients, solubilizers, dispersant, stabilizers, suspending agents, colorants and fragrances, etc.; for injections, buffers, preservatives, pain relief agents, solubilizers, isotonic agents, and stabilizers may be mixed and used; and for topical administration, bases, excipients, lubricants, and preservatives may be used.

In an embodiment, the pharmaceutical composition may further include a pharmaceutically acceptable excipient.

The dosage form of the pharmaceutical composition may be prepared in various ways by mixing with a pharmaceutically acceptable carrier as described above. For example, in the case of oral administration, the form of tablets, troches, capsules, elixirs, suspensions, syrups, and wafers may be used, and in the case of injections, the form of unit dose ampoules or multiple doses may be used. In addition, the formulation may be prepared in the form of solutions, suspensions, tablets, pills, capsules, sustained-release preparations, and the like.

Meanwhile, examples of suitable carriers, excipients and diluents for formulation include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, or mineral oil. In addition, fillers, anti-agglomeration agents, lubricants, wetting agents, flavoring agents, emulsifiers, and preservatives and the like may be further included.

The pharmaceutical composition may further include one or more other agents for treating a lung disease. In an embodiment, the other agent may be an anti-inflammatory agent or an immunosuppressive agent, but embodiments of the present disclosure are not limited thereto. In an embodiment, the other agent may be a therapeutic agent for lung disease, but embodiments of the present disclosure are not limited thereto.

The "anti-inflammatory agent" refers to a compound for the treatment of an inflammatory disease or condition associated therewith. Non-limiting examples of the anti-inflammatory agent are non-steroidal anti-inflammatory drugs (NSAID; e.g., aspirin, ibuprofen, naproxen, methyl salicylate, diflunisal, indomethacin, sulindac, diclofenac, ketoprofen, ketorolac, carprofen, fenoprofen, mefenamic acid, piroxicam, meloxicam, methotrexate, celecoxib, valdecoxib, parecoxib, etoricoxib, and nimesulide), corticosteroids (e.g., prednisone, betamethasone, budesonide, cortisone, dexamethasone, hydrocortisone, methylprednisolone, prednisolone, tramcinolone, and fluticasone), rapamycin (e.g., document [Migita et al., Clin. Exp. Immunol. (1997) 108:199-203]; [Migita et al., Clin. Exp. Immunol. (1996) 104:86-91]; [see Foroncewicz et al., Transpl. Int. (2005) 18:366-368), high-density lipoprotein (HDL) and HDL-cholesterol-raising compounds (e.g., Birjmohun et al. (2007) Arterioscler. Thromb. Vasc. Biol., 27:1153-1158]; [Nieland et al. (2007) J. Lipid Res., 48:1832-1845]; the use of rosiglitazone as an anti-inflammatory agent disclosed by [Bloedon et al. (2008) J. Lipid Res., Samaha et al. (2006) Arterioscler. Thromb. Vasc. Biol., 26:1413-1414], [Duffy et al. (2005) Curr. Opin. Cardiol., 20:301-306]), rho-kinase inhibitors (e.g., see document [Hu, E. (2006) Rec. Patents Cardiovasc. Drug Discov., 1:249-263]), antimalarial agents (e.g., hydroxychloroquine and chloroquine), acetaminophen, glucocorticoids, steroids, beta-agonists, anticholinergics, methyl xanthine, gold infusion (e.g., sodium orothiomalate), sulfasalazine, penicillamine, antiangiogenic agents, dapsone, psoralen, antiviral agents, statins (e.g., document [Paraskevas et al. (2007) Curr. Pharm. Des., 13:3622-36]; see [Paraskevas, K.I. (2008) Clin. Rheumatol. 27:281-287), and antibiotics (e.g., tetracycline). In certain embodiments, the anti-inflammatory agent is a statin or high-density lipoprotein (HDL) and a HDL-cholesterol-raising compound.

The "Immunosuppressant" and "immunosuppressive agent" include compounds or compositions that inhibit an immune response or symptoms associated therewith. Immunosuppressants include, but are not limited to, purine analogs (e.g., azathioprine), methotrexate, cyclosporine (e.g., cyclosporine A), cyclophosphamide, leflunomide, mycophenolate (mycophenolate mofetil), steroids (e.g., glucocorticoids, corticosteroids), methylprednisone, prednisone, nonsteroidal anti-inflammatory drugs (NSAIDs), chloroquine, hydroxychloroquine, chlorambucil, CD20 antagonists (e.g., rituximab, ocrelizumab), beltuzumab or ofatumumab), abatacept, TNF antagonists (e.g., infliximab, adalimumab, etanercept), macrolides (e.g., pimecrolimus, tacrolimus (FK506), and sirolimus), dihydroepiandrosterone, lenalidomide, CD40 antagonist (e.g., anti-CD40L antibody), avetimus sodium, BLys antagonist (e.g., anti-BLyS (e.g., belimumab)), dactinomycin, bucilamine, penicillamine, leflunomide, mercaptopurine, pyrimidine analogs (e.g., cytosine arabinoside), mizoribine, alkylating agents (e.g., nitrogen mustard, phenylalanine mustard, busulfan, and cyclophosphamide), folate antagonists (e.g., aminopterin and methotrexate), antibiotics (e.g., rapamycin, actinomycin D, mitomycin C, furamycin, and chloramphenicol), human IgG, anti-lymphocyte globulin (ALG), antibodies (e.g., anti-CD3 (OKT3), anti-CD4 (OKT4), anti-CD5, anti-CD7, anti-IL-2 receptors (e.g., daclizumab and basiliximab), anti-alpha/beta TCR, anti-ICAM-1, murononab-CD3, anti-IL-12, alemutuzumab and antibodies to immunotoxins), 1-methyltryptophan, and derivatives and analogs thereof. In certain embodiments, the immunosuppressant is selected from methotrexate, hydroxychloroquine, a CD20 antagonist (e.g., rituximab, ocrelizumab, veltuzumab or ofatumumab), abatacept, a TNF antagonist (e.g., infliximab, adali mumab, or etanercept), sirolimus, and a BLyS antagonist (e.g., anti-BLyS (e.g., belimumab)).

A "therapeutic agent for lung disease" includes a compound or composition that inhibits or treats symptoms associated with lung disease. As the lung disease therapeutic agent, any known material may be used. For example, the lung disease therapeutic agent may be roflumilast or the like.

The dosage and frequency of administration of the pharmaceutical composition is determined according to the type of drug as an active ingredient, along with several related factors such as the disease to be treated, the route of administration, the age, sex and weight of the patient, and the severity of the disease.

Since the pharmaceutical composition has excellent *in vivo* persistence and potency, the number and frequency of administration may be significantly reduced.

Another aspect provides a method of preventing or treating lung disease, including administering an effective amount of the GIP derivative, a pharmaceutically acceptable salt thereof, a solvate thereof, or a conjugate thereof, or a pharmaceutical composition, to a subject in need thereof.

The GIP derivative, the pharmaceutically acceptable salt thereof, the solvate thereof, the conjugate thereof, the pharmaceutical composition, and lung disease are the same as described above.

An "effective amount" or "pharmaceutically effective amount" refers to the amount or dose of the GIP derivative, a pharmaceutically acceptable salt thereof, a solvate thereof, or a conjugate thereof, or a combination thereof to provide the target effect in the patient under diagnosis or treatment when administered to a patient in single or multiple doses. An effective amount may be readily determined by the attending physician of ordinary skill in the art by using known techniques or by considering the results obtained under similar circumstances. When determining an effective amount for a patient, various unlimiting factors are considered by the attending physician, including: mammalian species; sizes, age, and general health conditions thereof; the specific disease or disorder involved; degree or severity of involvement of the disease or disorder; individual patient responses; the particular compound being administered; mode of administration; the bioavailability characteristics of the agent being administered; selected dosing regimen; use of concomitant medications; and other relevant circumstances.

The term "subject" refers to a subject in need of treatment for a disease, and, for example, mammals such as human or non-human primates, mice, rats, dogs, cats, horses, and cattle.

The term "administration" refers to introducing a given substance into a patient by any suitable method. The route of administration may be any general route capable of reaching the i*n vivo* target of the patient. The administration may be, for example, intraperitoneal administration, intravenous administration, intramuscular administration, subcutaneous administration, intradermal administration, oral administration, topical administration, intranasal administration, or rectal administration, but is not limited thereto.

The dosage may be prescribed in various ways depending on factors such as a formulation method, an administration mode, the age, weight, and sex of the patient, a pathological condition, food, an administration time, an administration route, an excretion rate, and a response sensitivity of the patient, and in consideration of these factors, those skilled in the art may appropriately adjust the dosage. The number of administration may be once a day or twice or more within the range of clinically acceptable side effects, the administration site may be one or two or more sites, the administration interval may be daily or at intervals of 2 to 5 days, and the number of total days of administration may range from 1 day to 30 days per treatment. If necessary, the same treatment can be repeated after an appropriate time. For animals other than humans, the dose is the same as that of a human per kg, or this dose is converted, for example, by the volume ratio (for example, average value) of the target animal and the organ (heart, etc.) of the human, and the converted dose is administered.

In the method, an effective amount of the GIP derivative, a pharmaceutically acceptable salt thereof, a solvate thereof, or a conjugate thereof may be administered simultaneously, separately, or sequentially with an effective amount of one or more other active ingredients. The one or more other active ingredients may be one or more other agents for treating a lung disease, but are not limited thereto.

Another aspect provides the use of the GIP derivative, a pharmaceutically acceptable salt thereof, a solvate thereof, or a conjugate thereof for use in the preparation of a medicament for the prevention or treatment of lung disease.

The GIP derivative, the pharmaceutically acceptable salt thereof, the solvate thereof, the conjugate thereof, and lung disease are the same as described above.

Each description and embodiment described herein is also applicable to each other description and embodiment. That is, all combinations of the various elements disclosed herein are within the scope of the present disclosure. In addition, it cannot be said that the scope of the present invention is limited by the specific descriptions described below.

### ADVANTAGEOUS EFFECTS OF DISCLOSURE

A GIP derivative or a long-acting conjugate thereof according to an aspect, suppresses inflammation of the lung, ameliorates pulmonary fibrosis by inhibiting epithelial-mesenchymal transition of alveolar epithelial cells, and has an effect of ameliorating emphysema, thus preventing or treating lung diseases such as COPD.

### BRIEF DESCRIPTION OF DRAWINGS

FIG. 1 shows a diagram showing the results of preparing a GIP derivative (SEQ ID NO: 11, 17, 21, and 24)-PEG-immunoglobulin Fc region conjugate and analyzing the same by SDS-PAGE.
FIG. 2A shows a graph showing the relative expression levels of inflammation-related genes IL-6, IL-12, IL-1β, and TNF-α after treatment with native GIP or long-acting GIP derivatives.
FIG. 2B shows a graph showing the concentrations (ng/mL) of the inflammation-related cytokines IL-6, IL-12/23 p40, IL-1β, and TNF-α after treatment with native GIP or long-acting GIP derivatives.
FIG. 3A shows a graph showing the relative expression levels of inflammation-related genes IL-6, IL-12, and TNF-α after treatment with native GIP or long-acting GIP derivatives.
FIG. 3B shows a graph showing the relative expression levels of EMT marker fibronectin, Col1α1, and Col3α1 after treatment with native GIP or long-acting GIP derivatives.
FIG. 4 shows results for confirming the degree of emphysema in a normal mouse control group, an excipient control group, a group administered with roflumilast, and a group administered with a long-acting GIP derivative.
FIG. 5A shows a graph showing lung weights 3 weeks after administration of a normal mouse control group, an excipient control group, a group administered with roflumilast, and a group administered with long-acting GIP derivative.
FIG. 5B shows a graph showing the relative expression levels of inflammatory cytokines IL-1β, IL-6, IL-12 and TNF-α in lung tissue in a normal mouse control group, an excipient control group, a group administered with roflumilast, and a group administered with long-acting GIP derivative.

### BEST MODE

Hereinafter, the present invention will be described in more detail through examples. However, these examples are for illustrative purposes only, and the scope of the present disclosure is not limited to these examples.

### Example 1: Preparation of GIP derivatives having activity with respect to GIP receptors

GIP derivatives showing activity on human GIP receptors were prepared and the sequences thereof are shown in Table 1 below.

**[Table 1]**

| SEQ ID NO: | Amino acid sequence |
|---|---|
| 1 | YAibEGT FISDY SIAMD AIAQQ DFVNW LLAQK PSSGA PPPSC |
| 2 | YAibEGT FISDY SIAMD AIAQQ DFVNW LLAGG PSSGA PPPSC |
| 3 | YAibEGT FISDY SIAibMD AIAQQ DFVNW LLAGG PSSGA PPPSC |
| 4 | YAibEGT FISDY SIYMD AIAQQ DFVNW LLAGG PSSGA PPPSC |
| 5 | YAibEGT FISDY SIQMD AIAQQ DFVNW LLAGG PSSGA PPPSC |
| 6 | YAibEGT FISDY SIYLD AIAQQ DFVNW LLAGG PSSGA PPPSC |
| 7 | YAibEGT FISDY SIYLD AQAQQ DFVNW LLAGG PSSGA PPPSC |
| 8 | YAibEGT FISDY SIYLD AQAAAib DFVNW LLAGG PSSGA PPPSC |
| 9 | YAibEGT FISDY SIYLD AQAAK DFVNW LLAGG PSSGA PPPSC |
| 10 | YAibEGT FISDY SIYLD AQAAK EFIAW LLAGG PSSGA PPPSC |
| 11 | YAibEGT FISDY SIAMD AIAQQ DFVNW LLAQK GKKND WKHNI TQC |
| 12 | YAibEGT FISDY SIYLD KQAAAib EFVNW LLAQK GKKND WKHNI TQC |
| 13 | YAibEGT FISDY SIYLD KQAAAib EFVNW LLAQK C |
| 14 | YAibEGT FISDY SIYLD AQAAAib EFVNW LLAQK C |
| 15 | YAibEGT FISDY SIAMD KIAQQ DFVNW LLAQK PSSGA PPPSC |
| 16 | YAibEGT FISDY SIAMD GIAQQ DFVNW LLAQK PSSGA PPPSC |
| 17 | YAibEGT FISDY SIALE KQAQQ DFVNW LLAGG PSSGA PPPSC |
| 18 | YAibEGT FISDY SIYLD KQAAQ EFVNW LLAQK PSSGA PPPSC |
| 19 | YAibEGT FISDY SIYLD KQAAAib EFVNW LLAibGH PSSGA PPPSC |
| 20 | YAibEGT FISDY SIYLD KQAAAib EFVNW LLAibGG PSSGA PPPSC |
| 21 | YAibEGT FISDY SIAibLD KQAAAib EFVNW LLAibGG PSSGA PPPSC |
| 22 | YAibEGT FISDY SIYLD KQAAAib EFVNW LLAGH PSSGA PPPSC |
| 23 | YAibEGT FISDY SIYLD KQAQK EFVNW LLAibGG PSSGA PPPSC |
| 24 | YAibEGT FISDY SIAibLD KQAAAib EFVNW LLAibGH PSSGA PPPSC |
| 25 | YAibEGT FISDY SIYLD KQAAAib EFVQW LlAibGG PSSGA PPPSC |
| 26 | YAibEGT FISDY SIYLD KQAAAib EFVQW LIAGH PSSGA PPPC |

The amino acid denoted by Aib in the sequences shown in Table 1 is Aib (aminoisobutyric acid), which is a non-natural amino acid.

The GIP derivative peptide is used as a GIP derivative having an amidated C-terminus if necessary.

### Example 2: Measurement of in vitro activity of GIP derivative

In order to measure the activity of the GIP derivative prepared in Example 1, a GIP receptor-transformed cell line was used to measure the cell activity *in vitro.* The cell line was transformed to express each human GIP receptor gene in Chinese hamster ovary (CHO), and is suitable for measuring the activity of GIP.

To measure the activity of the GIP derivative prepared in Example 1 in the human GIP receptor, human GIP was serially diluted from 16 nM to 0.000015 nM by 4 times, and the GIP derivative prepared in Example 1 was serially diluted from 16 nM to 0.000015 nM by 4 times. The culture medium was removed from the cultured CHO cells expressing the human GIP receptor, 5 µl of each serially diluted substance was added to the cells, and 5 µl of a buffer containing cAMP antibody was added thereto, followed by culturing at room temperature for 15 minutes. Then, 10 µl of a detection mix containing cell lysis buffer was added to lyse the cells, followed by reaction at room temperature for 90 minutes. The cell lysate after the reaction was completed, was applied to the LANCE cAMP kit (PerkinElmer, USA) to calculate EC₅₀ values through the accumulated cAMP, and then the values were compared with each other.

Relative titers of human GIP receptors compared to human GIP are shown in Table 2 below.

**[Table 2]**

| SEQ ID NO: | *In vitro* activity (%) of GIP derivatives compared to human native GIP in human GIP receptors |
|---|---|
| 1 | 49.3% |
| 2 | 13.9% |
| 3 | 15.6% |
| 4 | 10.5% |
| 5 | 12.1% |
| 6 | 17.4% |
| 7 | 19.6% |
| 8 | 1.9% |
| 9 | 2.0% |
| 10 | 13.9% |
| 11 | 121.9% |
| 12 | 49.2% |
| 13 | 31.1% |
| 14 | 17.4% |
| 15 | 31.1% |
| 16 | 11.3% |
| 17 | 111.4% |
| 18 | 13.6% |
| 19 | 66.7% |
| 20 | 75.3% |
| 21 | 122.7% |
| 22 | 71.6% |
| 23 | 87.6% |
| 24 | 183.0% |
| 25 | 78.3% |
| 26 | 66.7% |

### Example 3: Preparation of long-acting GIP conjugate

A long-acting conjugate including the GIP derivative prepared in Example 1 was prepared. For example, the GIP derivatives of SEQ ID NOS: 11, 17, 21 and 24 were each linked to the immunoglobulin Fc region via PEG, a non-peptidic polymer, respectively.

The specific long-acting conjugate preparation process is as follows, and the same process was repeated to prepare the GIP derivative conjugates of SEQ ID NOS: 11, 17, 21 and 24. For pegylation at the N-terminus of the immunoglobulin Fc region, the reaction was cause to occur at a molar ratio of the immunoglobulin Fc region and MAL-10K PEG-ALD (10 kDa PEG including maleimide and propionaldehyde groups, NOF, JAPAN) of 1:1-2, a total protein concentration of 40 mg/ml to 60 mg/ml, pH 6.0-pH 6.5, 4 °C -8 °C for about 3-4 hours. At this time, sodium cyanoborohydride (NaCNBH₃) reducing agent was added for the reaction, and the reaction solution was purified using a CaptoQ ImpRes (GE Healthcare Life Science, USA) column to purify a single pegylated immunoglobulin Fc region.

In order to bind the purified single pegylated immunoglobulin Fc region to the GIP derivative, the reaction was derived to occur at the molar ratio of the single pegylated immunoglobulin Fc region to the GIP derivatives (SEQ ID NOS: 11, 17, 21 and 24) is 1:1 to 3, at a total protein concentration of 0.1 mg/ml to 0.5 mg/ml in a buffer containing isopropanol at 4 °C to 8 °C for about 14 to 18 hours. The reaction solution was subjected to a Source 15ISO (GE Healthcare Life Science, USA) column to purify a conjugate in which each of GIP derivatives (SEQ ID NOS: 11, 17, 21 and 24) was covalently linked to the immunoglobulin Fc region by PEG.

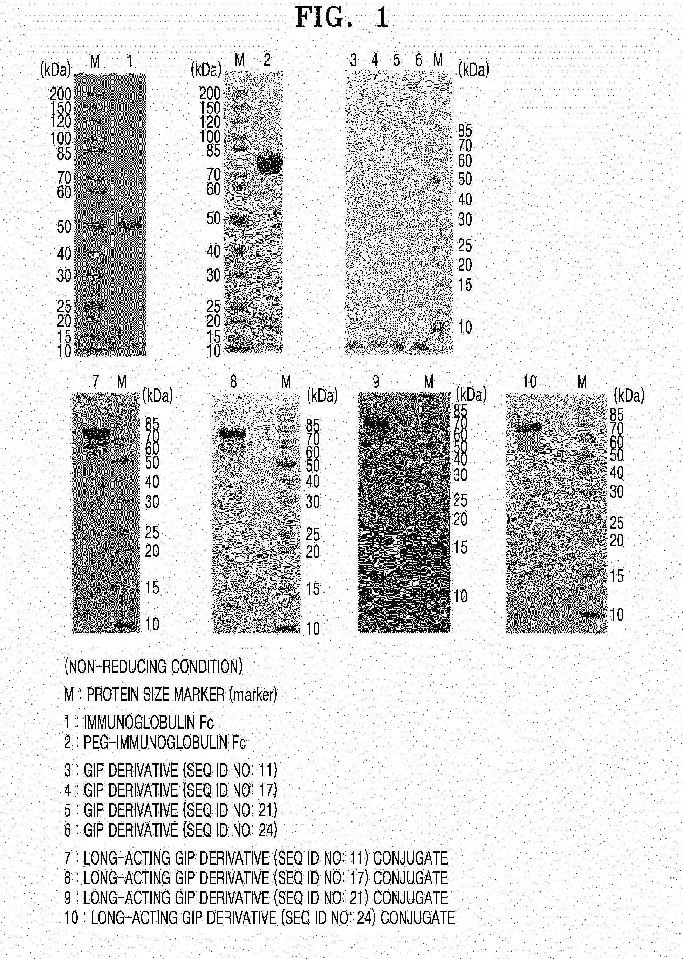

As a result, it was confirmed that a purified GIP derivative of SEQ ID NO: 11-PEG-immunoglobulin Fc region conjugate, a purified GIP derivative of SEQ ID NO: 17-PEG-immunoglobulin Fc region conjugate, a purified GIP derivative of SEQ ID NO: 21-PEG-immunoglobulin Fc region conjugate, and a purified GIP derivative of SEQ ID NO: 24-PEG-immunoglobulin Fc region conjugate were prepared with a purity of 90% or more, and the results of SDS-PAGE analysis are shown in FIG. 1.

### Example 4: Measurement of in vitro activity of long-acting GIP conjugate

In order to measure the activity of the long-acting GIP conjugate prepared in Example 3, as in the same manner as in Example 2, a method of measuring cell activity *in vitro* using a GIP receptor-transformed cell line was used.

For example, to measure the activity of the long-acting GIP conjugate in the human GIP receptor, human GIP was serially diluted from 16 nM to 0.000015 nM by 4 times, and the long-acting GIP conjugate was serially diluted from 50 nM to 0.000048 nM by 4 times. The culture medium was removed from the cultured CHO cells expressing the human GIP receptor, 5 µl of each serially diluted substance was added to the cells, and 5 µl of a buffer containing cAMP antibody was added thereto, followed by culturing at room temperature for 15 minutes. Then, 10 µl of a detection mix containing cell lysis buffer was added to lyse the cells, followed by reaction at room temperature for 90 minutes. The cell lysate after the reaction was completed, was applied to the LANCE cAMP kit (PerkinElmer, USA) to calculate EC₅₀ values through the accumulated cAMP, and then the values were compared with each other.

Relative titers of human GIP receptors compared to human GIP are shown in Table 3 below.

**[Table 3]**

| SEQ ID NO: | *In vitro* activity (%) of long-acting GIP conjugates compared to human native GIP in human GIP receptors |
|---|---|
| 11 | 84.8% |
| 17 | 153.2% |
| 21 | 148.5% |
| 24 | 123.3% |

The example shows that the GIP derivative of the present disclosure has the activity of native GIP, and especially when prepared as a long-acting conjugate, the GIP derivative exhibits an activity equal to or higher than that of native GIP and has an increased half-life, thereby showing excellent properties as a drug.

### Example 5: Confirmation of anti-inflammatory in-vitro effect of long-acting GIP conjugate

To confirm the anti-inflammatory effect of the long-acting GIP derivative on COPD *in vitro,* a human monocyte/macrophage cell line, THP-1, was used. Macrophages are known to induce the progression of inflammation by secreting cytokines and chemokines at the initial stage of infection and mobilizing other immune cells.

The THP-1 cell line was cultured in a RPMI 1640 medium supplemented with 10% fetal bovine serum (FBS), 100 µg/mL streptomycin, 100 U/mL penicillin, and 0.05 mM β-mercaptoethanol, at a temperature of 37 °C in 5% carbon dioxide conditions. An inflammatory response was induced by adding 1 µg/mL of lipopolysaccharide (LPS) to the cell line, and the effect on the inflammatory response by LPS when treated with native GIP and long-acting GIP derivatives was confirmed at the mRNA level. The native GIP was added at the concentration 10 µM, and the long-acting GIP derivative was diluted at a concentration of 1 or 10 µM and added. As the long-acting GIP derivative, the long-acting GIP conjugate (SEQ ID NO: 17) prepared in Example 3 was used.

RNA was isolated from the THP-1 cell line after the treatment was completed by using the RNeasy Mini Kit (Qiagen, US), and cDNA was synthesized using the iScript^{™} cDNA Synthesis Kit (Bio-rad, US). Regarding the synthesized cDNA, the expression level of inflammation-related genes was confirmed by using the QuantStudio 6 Flex Real-Time PCR System (Applied Biosystems, US). Delta Delta Ct method was used, and beta-actin was used as a housekeeping gene. IL-6, IL-12, IL-1β, and TNF-α were identified as inflammation-related genes.

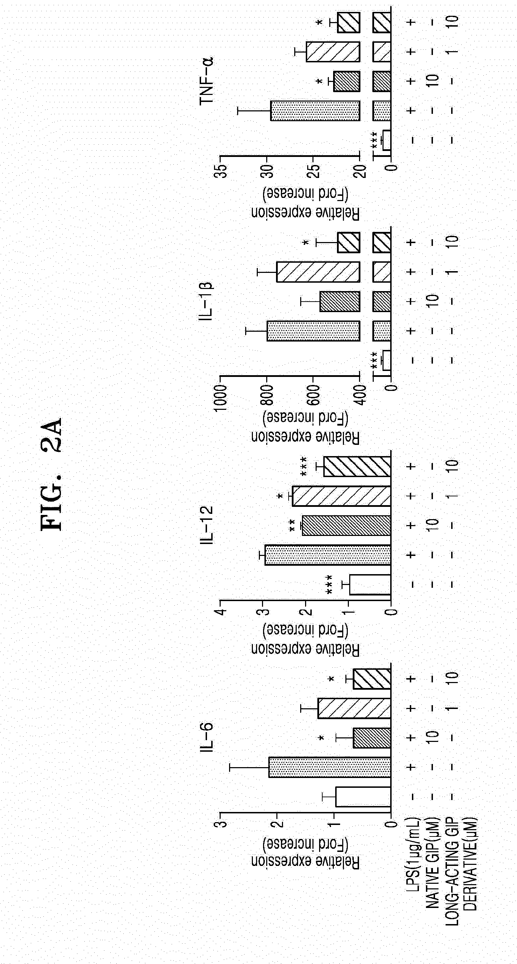

FIG. 2A shows a graph showing the relative expression levels of inflammation-related genes IL-6, IL-12, IL-1β, and TNF-α after treatment with native GIP or long-acting GIP derivatives.

As shown in FIG. 2A, it was confirmed that the expression level of the inflammation-related genes was increased by induced inflammatory response when treated with LPS, and the expression thereof was decreased when treated with native GIP and long-acting GIP derivatives. It was confirmed that these results were dependent on the concentration of the long-acting GIP derivative conjugate.

Additionally, in order to measure the concentrations of the inflammation-related cytokines IL-6, IL-12/23 p40, IL-1β, and TNF-α in the medium of the THP-1 cell line, 150 nm of phorbol 12-myristate 13-acetate (PMA) and 0.1 µg/mL of LPS were added thereto to induce an inflammatory reaction, and the cells were treated with the native GIP and the long-acting GIP derivative: the native GIP was used at a concentration of 1 µM and the long-acting GIP derivative was diluted to a concentration of 0.1 µM or 1 µM. As the medium, Human IL-6 ELISA Kit (Abcam, US), Human IL-12/23 p40 ELISA Kit (Abcam, US), Human IL-1beta ELISA Kit (Abcam, US), and Human TNF alpha ELISA Kit (Abcam, US)were used for quantification.

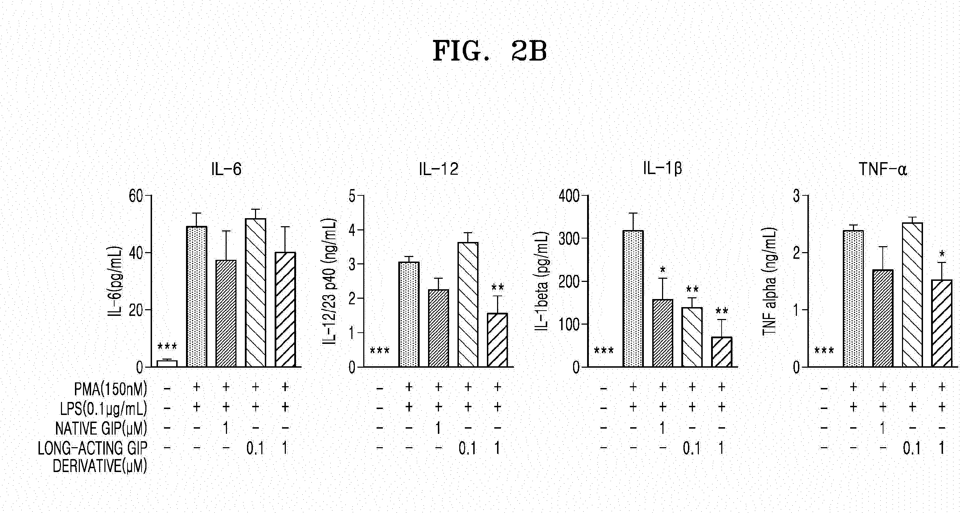

FIG. 2B shows a graph showing the concentrations (ng/mL) of the inflammation-related cytokines IL-6, IL-12/23 p40, IL-1β, and TNF-α after treatment with native GIP or long-acting GIP derivatives.

As shown in FIG. 2B, it was confirmed that the concentration of inflammatory cytokines in the medium was increased when treated with PMA and LPS, and decreased when treated with native GIP and long-acting GIP derivatives.

Therefore, it can be seen that the long-acting GIP derivatives acted directly on macrophages to show anti-inflammatory effects that block the inflammatory responses induced by PMA and LPS. It can be inferred from the results of native GIP that the anti-inflammatory effect is due to the action of GIP.

### Example 6: Confirmation of in-vitro effect of ameliorating inflammation and fibrosis of long-acting GIP conjugate

The A549 cell line, a human alveolar epithelial cell, was used to confirm the inflammatory and fibrosis amelioration effect of long-acting GIP derivatives on COPD *in vitro.* The purpose of this experiment was to determine the effect of alveolar epithelial cells on epithelial mesenchymal transition (EMT), which is known to be important in the pulmonary fibrosis process.

The A549 cell line was cultured in a F-12K medium supplemented with 10% fetal bovine serum (FBS), 100 µg/mL streptomycin, and 100 U/mL penicillin at a temperature of 37 °C and in 5% carbon dioxide conditions. Cell lines were sequentially treated with TGF-β1 and LPS to induce inflammatory responses and EMT. The native GIP was added at the concentration 10 µM, and the long-acting GIP derivative was diluted at a concentration of 1 or 10 µM and added. As the long-acting GIP derivative, the long-acting GIP conjugate (SEQ ID NO: 17) prepared in Example 3 was used.

RNA was isolated from the A549 cell line after the treatment was completed by using the RNeasy Mini Kit (Qiagen, US), and cDNA was synthesized using the iScript^{™} cDNA Synthesis Kit (Bio-rad, US). Regarding the synthesized cDNA, the expression level of inflammation and EMT-related genes was confirmed by using the QuantStudio 6 Flex Real-Time PCR System (Applied Biosystems, US). Delta Delta Ct method was used, and beta-actin was used as a housekeeping gene. IL-6, IL-12, and TNF-α were identified as inflammation-related genes, and fibronectin, Col1α1 (collagen, type I, alpha 1) and Col3α1 (collagen, type III, alpha 1) were identified as EMT markers.

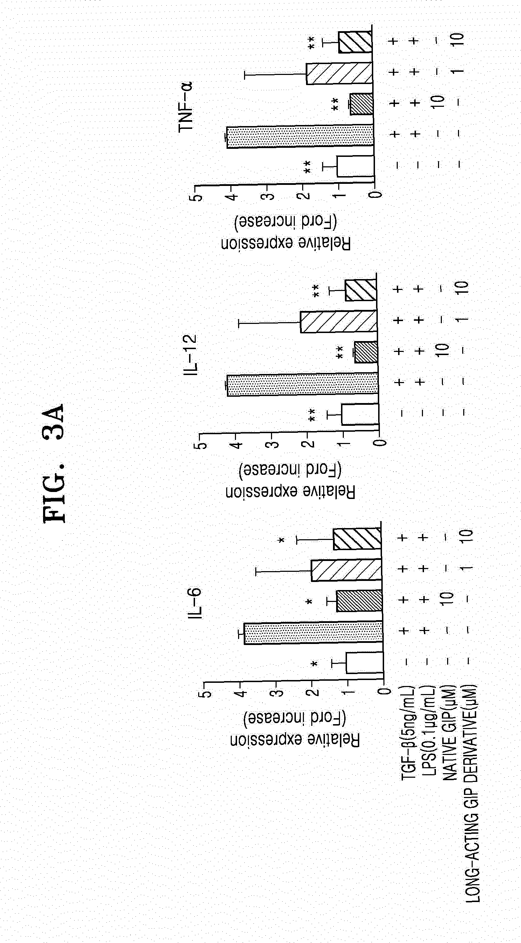

FIG. 3A is a graph showing the relative expression levels of inflammation-related genes IL-6, IL-12, and TNF-α after treatment with native GIP or long-acting GIP derivatives.

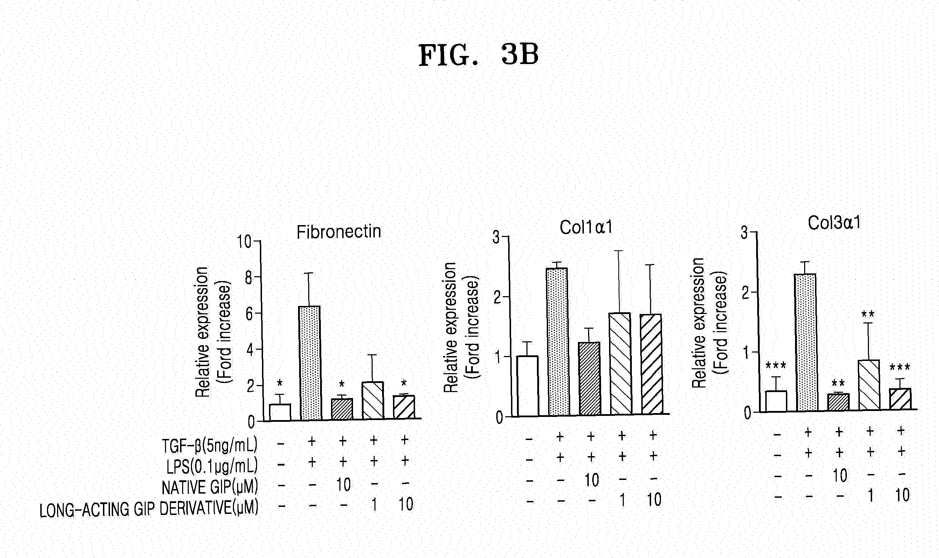

FIG. 3B is a graph showing the relative expression levels of EMT marker fibronectin, Col1α1, and Col3α1 after treatment with native GIP or long-acting GIP derivatives.

As shown in FIG. 3A, it was confirmed that the expression level of the inflammation-related genes was increased by induced inflammatory response when treated with TGF-β and LPS, and the expression thereof was significantly decreased when treated with native GIP and long-acting GIP derivatives.

As shown in FIG. 3B, when treated with TGF-β and LPS, the expression of EMT markers was increased. However, when treated with native GIP and long-acting GIP derivatives, the expression thereof as decreased.

Therefore, it was confirmed that the long-acting GIP derivative could ameliorate chronic bronchitis and pulmonary fibrosis by inhibiting inflammation and epithelial-mesenchymal transition (EMT) of alveolar epithelial cells.

### Example 7: Confirmation of COPD in-vivo effect of long-acting GIP derivatives

In addition to the *in-vitro* effect confirmed in the above example, it was tested whether the therapeutic effect of COPD, a representative lung disease and disease caused by lung inflammation, could be exhibited *in-vivo.* As the CDP animal model, mice administered with elastase (ELA) were used.

In detail, ELA 0.2U/mouse was administered to C57BL/6 mice (DBL Co., Ltd,, Korea) to prepare COPD animal model mice, which were then divided into an excipient control, a group administered with long-acting GIP derivative (3.16 mg/kg, Q2D, subcutaneously), and a group administered with roflumilast (10 mg/kg, QD, oral), which is an anti-inflammatory agent and a treatment for COPD. For each group, corresponding administration was repeatedly performed for three weeks. As the long-acting GIP derivative, the long-acting GIP conjugate (SEQ ID NO: 17) prepared in Example 3 was used.

Then, the lung tissue of each mouse was taken by an autopsy, and the degree of emphysema of the lung tissue was evaluated through H&E staining. In addition, the lung weight was measured, and the degree of change in the expression of inflammatory cytokines in the lung tissue according to administration of an excipient, a long-acting GIP derivative, or roflumilast was confirmed through qPCR to evaluate the effect on inflammation.

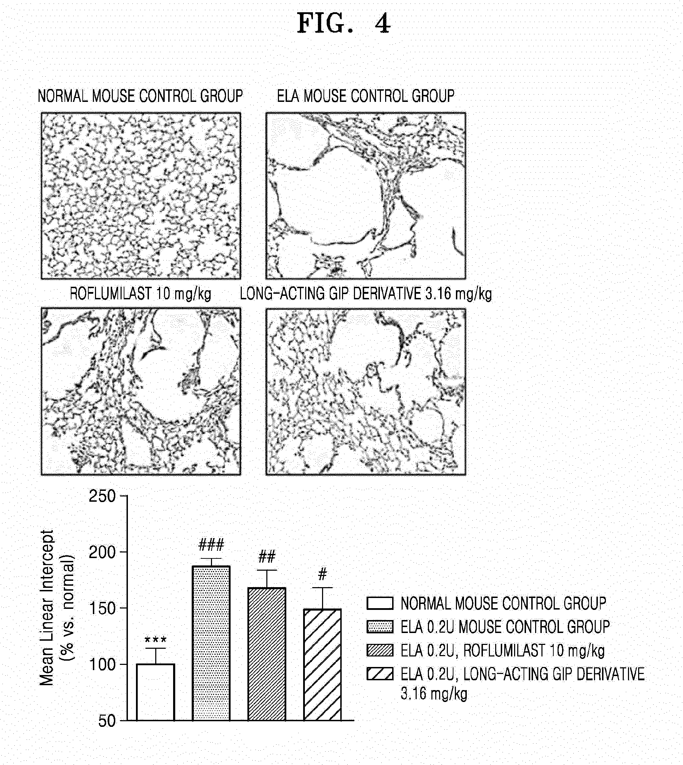

FIG. 4 shows results for confirming the degree of emphysema in a normal mouse control group, an excipient control group, a group administered with roflumilast, and a group administered with a long-acting GIP derivative.

As shown in FIG. 4, when the long-acting GIP derivative was repeatedly administered, excellent emphysema amelioration efficacy was confirmed compared to the excipient control group and the roflumilast administration group.

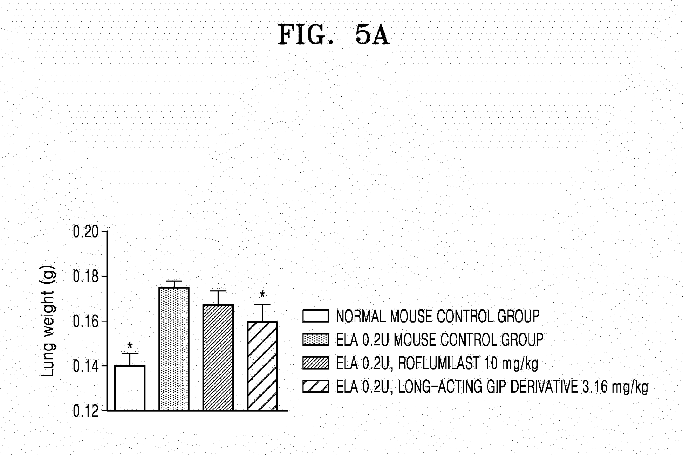

FIG. 5A shows a graph showing lung weights 3 weeks after administration of a normal mouse control group, an excipient control group, a group administered with roflumilast, and a group administered with long-acting GIP derivative.

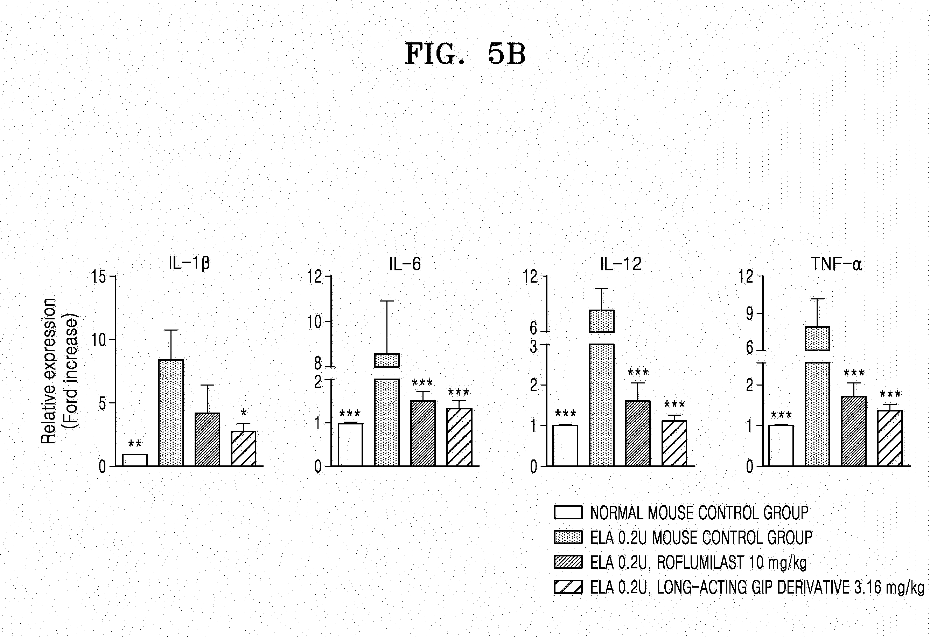

FIG. 5B shows a graph showing the relative expression levels of inflammatory cytokines IL-1β, IL-6, IL-12 and TNF-α in lung tissue in a normal mouse control group, an excipient control group, a group administered with roflumilast, and a group administered with long-acting GIP derivative.

As shown in FIG. 5A, the degree of inflammation can be indirectly confirmed through the weight of the lung. When the long-acting GIP derivative was administered, the lung weight was significantly reduced compared to the excipient control group and the roflumilast administration group.

In addition, as shown in FIG. 5B, the gene expression of inflammatory cytokines in the lung tissue showed a tendency to significantly decrease in the group administered with the long-acting GIP derivative compared to the control group.

Therefore, it was confirmed *in-vivo* that the long-acting GIP derivative can improve lung inflammation and emphysema.

As such, it was confirmed that the long-acting GIP derivative or the conjugate thereof acts on macrophages to inhibit inflammation-related cytokines IL-6, IL-12/23 p40, IL-1β, and TNF-α, thereby exhibiting an anti-inflammatory effect, to inhibit inflammation-related genes IL-6, IL-12, and TNF-α in alveolar epithelial cells, thereby exhibiting anti-inflammatory effects, and to inhibit epithelial-mesenchymal transition (EMT) of alveolar epithelial cells, thereby ameliorating pulmonary fibrosis. In addition, it was confirmed that even *in vivo,* the long-acting GIP derivative ameliorates lung inflammation and emphysema. This indicates that the long-acting GIP derivative can be used as a treatment for lung diseases such as pneumonia, alveolitis, asthma, pulmonary fibrosis, emphysema, chronic bronchitis, COPD, and COVID-19.

## Claims

1. A pharmaceutical composition for the prevention or treatment of lung disease, comprising:
a pharmaceutically acceptable excipient and a peptide comprising the amino acid sequence of any one of SEQ ID NOS: 1 to 26 in a pharmaceutically effective amount.

2. The pharmaceutical composition of claim 1, wherein the peptide is in the form of a long-acting conjugate, and the long-acting conjugate is represented by Formula 1:
[Formula 1] X-L-F
where X is a peptide comprising the amino acid sequence of any one of SEQ ID NOS: 1 to 26;
L is a linker comprising an ethylene glycol repeating unit,
F is an immunoglobulin Fc region,
- represents a covalent bond linkage between X and L and between L and F.

3. The pharmaceutical composition of claim 1 or claim 2, wherein the peptide is amidated at a C-terminus thereof.

4. The pharmaceutical composition of claim 1 or claim 2, wherein the peptide comprises an amino acid sequence selected from the group consisting of SEQ ID NOS: 11, 17, and 19 to 26.

5. The pharmaceutical composition according to claim 4, wherein the peptide comprises an amino acid sequence selected from the group consisting of SEQ ID NOS: 11, 17, 21 and 24.

6. The pharmaceutical composition according to claim 2, wherein the formula weight of the ethylene glycol repeating unit moiety in L is in the range of 1 kDa to 100 kDa.

7. The pharmaceutical composition of claim 2, wherein F is an IgG Fc region.

8. The pharmaceutical composition of claim 1 or claim 2, wherein the pharmaceutical composition has one or more of the following effects:
(i) inhibition of lung inflammation;
(ii) inhibition of epithelial-mesenchymal transition (EMT) of alveolar epithelial cells; and
(iii) amelioration of emphysema.

9. The pharmaceutical composition of claim 1 or claim 2, wherein the lung disease is one or more selected from interstitial lung disease, progressive fibrotic interstitial lung disease, idiopathic interstitial pneumonia, non-specific interstitial pneumonia, pulmonary fibrosis, interstitial pulmonary fibrosis, idiopathic pulmonary fibrosis, alveolitis, pneumonia, emphysema, bronchitis, asthma, combined pulmonary fibrosis and emphysema (CPFE), chronic obstructive pulmonary disease (COPD), and coronavirus disease.

10. The pharmaceutical composition of claim 9, wherein the coronavirus disease is coronavirus infection-19 (COVID-19).
